# EUROPEAN PATENT APPLICATION

(11) **EP 4 331 608 A1**
(43) Date of publication of application: **06.03.2024**
(21) Application number: 23173354.4
(22) Date of filing: 30.09.2021
(51) Int. Cl.: A61K 47/54, C12N 15/113, A61P 3/06

(54) **TARGET LIGAND**

(30) Priority: 30.09.2020 CN 202011061038; 05.01.2021 CN 202110008013; 13.04.2021 CN 202110397429
(62) Divisional of application: 21874577.6
(71) Applicant: Nanopeptide (Qingdao) Biotechnology Ltd., Qingdao, Shandong 266199 (CN)
(72) Inventor: LIU, Zhaogui, Qingdao, 266199 (CN); CHEN, Ping, Qingdao, 266199 (CN); ZHANG, Jieting, Qingdao, 266199 (CN); WANG, Rui, Qingdao, 266199 (CN); XU, Juan, Qingdao, 266199 (CN); FU, Zhongguo, Qingdao, 266199 (CN); ZHANG, Hailin, Qingdao, 266199 (CN); CHEN, Pu, Qingdao, 266199 (CN)
(74) Representative: Zaboliene, Reda

(57) **Abstract**

The present disclosure relates to the technical field of genetic engineering, in particular to a N-acetyl-galactosamine (GalNAc) target ligand. The target ligand provided by the present disclosure forms a siRNA conjugate with a specific small interfering RNA sequence, which targets to an angiopoietin like 3 (ANGPTL3), and reduce the expression quantity of an ANGPTL3 protein by degrading a transcript of an *ANGPTL3* gene in a cell. Therefore, the siRNA conjugate formed by the target ligand provided by the present disclosure may be used to prevent and/or treat a dyslipidemia disease.

## Description

### Cross-Reference to Related Application

The present application is a divisional application, the parent application is an application for Patent Cooperation Treaty (PCT) to enter the national phase in China, the application number of the parent application is 2021800229023, the international application date of PCT is September 30, 2021, the date of entering the national phase in China is October 18, 2022, and the invention title of the parent application is "siRNA of angiopoietin-like 3 and use thereof".

### Technical Field

The present disclosure relates to the technical field of genetic engineering, in particular to a new compound that may be used as a target ligand.

### Background

Hyperlipidemia, also known as dyslipidemia, is a systemic disease with abnormal fat metabolism or operation, which makes plasma lipids higher than a normal value. The clinical manifestations of the dyslipidemia mainly include two aspects: (1) xanthoma caused by lipid deposition in dermis; and (2) atherosclerosis caused by the lipid deposition in vascular endothelium, generating a coronary heart disease and a peripheral vascular disease and the like. According to the survey, about 10% to 20% of adults have the elevated blood total cholesterol (TC) or triglycerides (TG), and even nearly 10% of children have the elevated blood lipids. Existing drugs for the dyslipidemia mainly include statins, cholesterol absorption inhibitors, resins, probucol, fibrates, niacin and derivatives thereof.

The angiopoietin like protein 3 (ANGPTL3, NM_014495.4) is a secreted protein mainly expressed in a liver cell. It is indicated from existing researches that ANGPTL3 is a key regulatory factor of low-density lipoprotein cholesterol (LDL-C), high-density lipoprotein cholesterol (HDL-C) and triglyceride metabolism, and has a variety of potential action nodes. The loss of function mutation of ANGPTL3 may lead to the reduction of LDL-C, very low-density lipoprotein cholesterol (VLDL-C), HDL-C and triglyceride (TG), thus the risk of cardiovascular diseases based on genome wide association study (GWAS) is reduced, and there are no known adverse phenotypes of genetic defects. Therefore, the inhibition of the activity of ANGPTL3 may effectively prevent or treat the dyslipidemia.

Most of existing technologies use an ANGPTL3 antibody to inhibit its activity.

A Chinese patent with the application number of CN201280038908.0 discloses a completely humanized antibody or an antigen-binding fragment of a human antibody, it specifically binds to a human angiopoietin-like protein 3 (hANGPTL3) and inhibits or interferes at least one of its activities. The human anti-hANGPTL3 antibody may be used to treat diseases or disorders related to ANGPTL3, such as hyperlipidemia, hyperlipoproteinemia and dyslipidemia, including hypertriglyceridemia, hypercholesterolemia, chylomicroxemia and the like.

A Chinese patent with the application number of CN201780026147. X discloses a method for treating a patient suffered from familial hypercholesterolemia, including HeFH and HoFH. By administering a therapeutically effective amount of the specific ANGPTL3-binding antibody or its antigen-binding fragment, it is combined with other drugs, and at least one lipid parameter of the patient is reduced. It may be used to treat hypercholesterolemia, hyperlipidemia, hyperlipoproteinemia and dyslipidemia, including the hypertriglyceridemia and the chylomicroxemia.

Compared with antibodies, the siRNA drug has the advantages of rich candidate targets, short research and development cycle, and high clinical development success rate. Therefore, it has the more advantages in the use of drugs for chronic diseases. However, the defects of the siRNA drug itself (such as: poor stability, and difficulty in transmembrane) limit its clinical applications. The structural transformation of siRNA or the construction of conjugates may improve the drug ability of siRNA to a certain extent. To explore constituent molecules of the conjugates constructed is the only way which must be passed to develop the siRNA drug for the dyslipidemia, and is also an urgent problem to be solved.

### Summary

The present disclosure aims to solve at least one of technical problems in a related technology to a certain extent. For this reason, a purpose of the present disclosure is to provide a siRNA for inhibiting expression of ANGPTL3. The inventor of the present disclosure targets to *ANGPTL3* by designing an appropriate specific small interfering RNA sequence and combining it with a target ligand to form a siRNA conjugate, and reduce the expression of an ANGPTL3 protein by degrading a transcript of an *ANGPTL3* gene in a cell. Therefore, the siRNA provided in the present disclosure may be used to prevent and/or treat a dyslipidemia disease.

For this reason, on the one hand, the present disclosure provides a siRNA. According to an embodiment of the present disclosure, the siRNA includes a sense chain and an antisense chain, and the antisense chain includes a complementary region complementary-paired to the sense chain, herein the sense chain is selected from a nucleotide sequence that is not more than 5 nucleotides different from a nucleotide sequence of each chain in SEQ ID NO: 1-SEQ ID NO: 154, and the antisense chain is selected from a nucleotide sequence that is not more than 5 nucleotides different from a nucleotide sequence of each chain in SEQ ID NO: 155-SEQ ID NO: 308.

The inventor of the present disclosure specifically reduces the synthesis of ANGPTL3 by the liver cell by designing the appropriate small interfering RNA (siRNA) sequence, while the off-target effect is avoided. siRNA, by forming a RNA-induced silencing complex (RISC), is complementary-paired with a mRNA sequence of a target gene (*ANGPTL3* gene) to degrade mRNA of the target gene so as to inhibit the expression of the target gene, and then reduce the levels of LDL-C, VLDL-C, HDL-C and TG

The siRNA according to an embodiment of the present disclosure may also have at least one of the following additional technical features.

The present disclosure further provides a siRNA, and the siRNA is selected from any pair of siRNA in any one of the following groups.
(1) It may specifically target to the 60-80-th nucleotides of the *ANGPTL3*gene sequence; preferably, the sense chain of the siRNA is selected from SEQ ID NO: 10, and the antisense chain is selected from SEQ ID NO: 165.
(2) It may specifically target to the 107-133-th nucleotides of the *ANGPTL3*genesequence; preferably, the sense chain of the siRNA is selected from SEQ ID NO: 17, and the antisense chain is selected from SEQ ID NO: 171, or the sense chain of the siRNA is selected from SEQ ID NO: 18, and the antisense chain is selected from SEQ ID NO: 172.
(3) It may specifically target to the 163-187-th nucleotides of the *ANGPTL3*genesequence; preferably, the sense chain of the siRNA is selected from SEQ ID NO: 19, and the antisense chain is selected from SEQ ID NO: 173.
(4) It may specifically target to the 304-388-th nucleotides of the *ANGPTL3*genesequence, and preferably, it may specifically target to the 304-359-th nucleotides of the ANGPTL3 sequence; more preferably, the sense chain of the siRNA is selected from SEQ ID NO: 27, and the antisense chain is selected from SEQ ID NO: 181,
   or, the sense chain of the siRNA is selected from SEQ ID NO: 29, and the antisense chain is selected from SEQ ID NO: 183,
   or, the sense chain of the siRNA is selected from SEQ ID NO: 31, and the antisense chain is selected from SEQ ID NO: 185,
   or, the sense chain of the siRNA is selected from SEQ ID NO: 32, and the antisense chain is selected from SEQ ID NO: 186,
   or, the sense chain of the siRNA is selected from SEQ ID NO: 35, and the antisense chain is selected from SEQ ID NO: 189,
   or, the sense chain of the siRNA is selected from SEQ ID NO: 36, and the antisense chain is selected from SEQ ID NO: 190.
(5) It may specifically target to the 430-459-th nucleotides of the *ANGPTL3*genesequence; preferably, the sense chain of the siRNA is selected from SEQ ID NO: 43, and the antisense chain is selected from SEQ ID NO: 197,
   or, the sense chain of the siRNA is selected from SEQ ID NO: 44, and the antisense chain is selected from SEQ ID NO: 198.
(6) It may specifically target to the 1360-1430-th nucleotides of the *ANGPTL3*genesequence, and preferably, it may specifically target to the 1397-1430-th nucleotides of the *ANGPTL3*genesequence; more preferably, the sense chain of the siRNA is selected from SEQ ID NO: 145, and the antisense chain is selected from SEQ ID NO: 299,
   or, the sense chain of the siRNA is selected from SEQ ID NO: 150, and the antisense chain is selected from SEQ ID NO: 304,
   or, the sense chain of the siRNA is selected from SEQ ID NO: 151, and the antisense chain is selected from SEQ ID NO: 305,
   or, the sense chain of the siRNA is selected from SEQ ID NO: 152, and the antisense chain is selected from SEQ ID NO: 306,
   or, the sense chain of the siRNA is selected from SEQ ID NO: 154, and the antisense chain is selected from SEQ ID NO: 308.

According to an embodiment of the present disclosure, the siRNA includes at least one modified nucleotide.

Optionally, the modified nucleotide is selected from at least one of the following:
a 5'-thiophosphate based nucleotide, a 5-methylcytosine nucleotide, a 2'-O-methyl modified nucleotide, a 2'-O-2-methoxyethyl modified nucleotide, a 2'-fluoro modified nucleotide, a 3'-nitrogen substituted modified nucleotide, a 2'-deoxy-2'-fluoro modified nucleotide, a 2'-deoxy modified nucleotide, a locked nucleotide, a de-base nucleotide, a 2'-amino modified nucleotide, a morpholino nucleotide, a polypeptide nucleotide, an amino phosphate, and a nucleotide including a non-natural base.

According to an embodiment of the present disclosure, the length of the complementary region is at least 17 bp.

Optionally, the length of the complementary region is 18-21 bp.

Optionally, the length of the complementary region is 19 bp.

According to an embodiment of the present disclosure, the lengths of the sense chain and the antisense chain in the siRNA are not more than 25 bp.

Optionally, the lengths of the sense chain and the antisense chain in the siRNA are 18-25 bp.

Optionally, the lengths of the sense chain and the antisense chain in the siRNA are 21 bp.

According to an embodiment of the present disclosure, the bases in the sense chain and the antisense chain of the siRNA may be complementary-paired one-to-one, or may be dislocated for several bases, but have at least 17 bp of the complementary region.

On the other hand, the present disclosure provides a siRNA conjugate, and the siRNA conjugate includes the previously described siRNA and a target ligand, herein the siRNA is covalently linked with the target ligand.

Preferably, the target ligand is linked to the sense chain in the siRNA.

More preferably, the target ligand is linked with a 5'-end of the sense chain in the siRNA by a thiophosphate bond.

According to an embodiment of the present disclosure, the target ligand includes at least one N-acetyl-galactosamine (GalNAC).

According to an embodiment of the present disclosure, the target ligand is a GalNAC target compound.

According to an embodiment of the present disclosure, the GalNAC target compound is 1043, 1046 and 1048, and its structure is shown as follows:

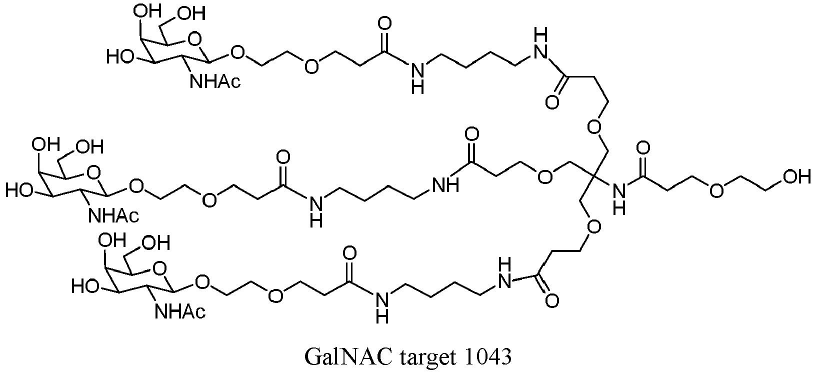

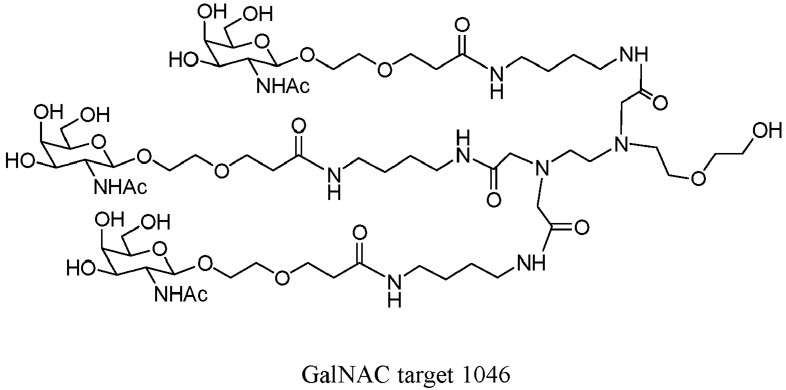

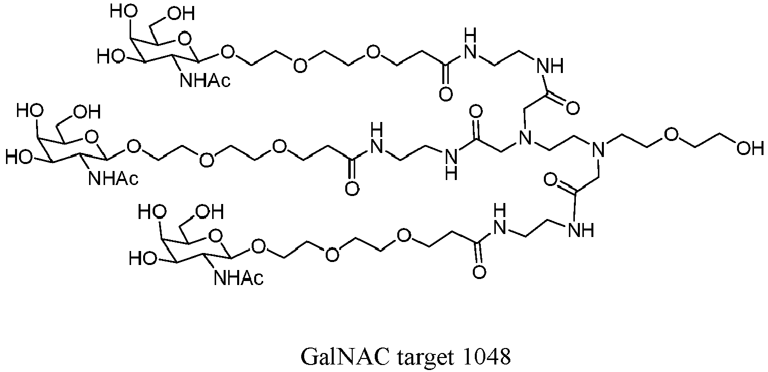

According to an embodiment of the present disclosure, the target ligand is linked to the sense chain in the siRNA.

On the other hand, the present disclosure provides a pharmaceutical composition. According to an embodiment of the present disclosure, the pharmaceutical composition includes the previously described siRNA and/or the previously described siRNA conjugate, and optionally, the pharmaceutical composition further includes a pharmaceutically acceptable excipient.

Therefore, the pharmaceutical composition according to the embodiment of the present disclosure may be used to inhibit the synthesis of ANGPTL3 by the cells, thereby the levels of LDL-C, VLDL-C, HDL-C and TG are reduced, as to prevent and/or treat hyperlipidemia and hypertriglyceridemia.

On the other hand, the present disclosure provides a compound.

The compound has any one of the following structures:

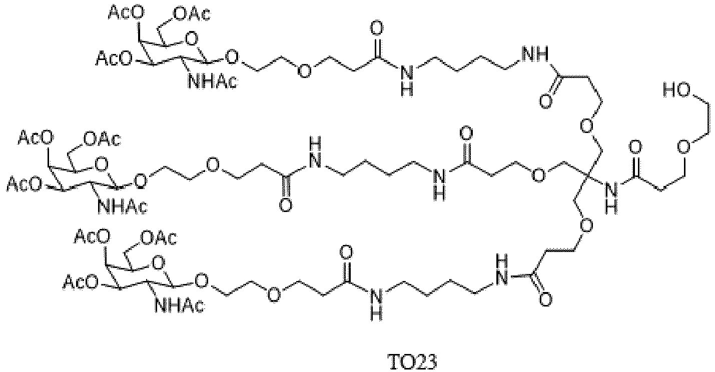

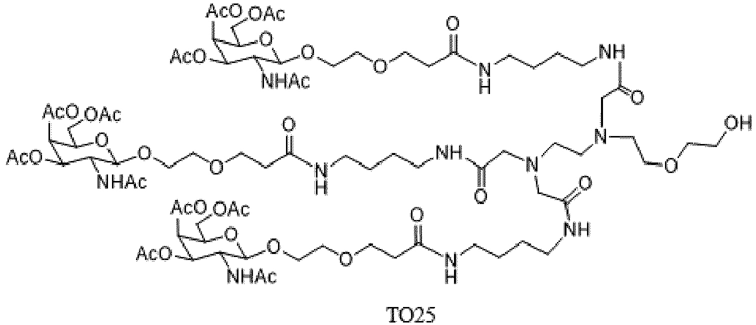

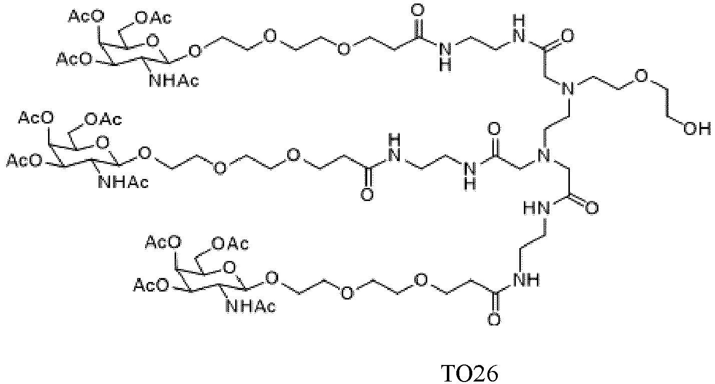

The structures of the above compounds obtained after a hydroxyl protecting group is removed are as follows:

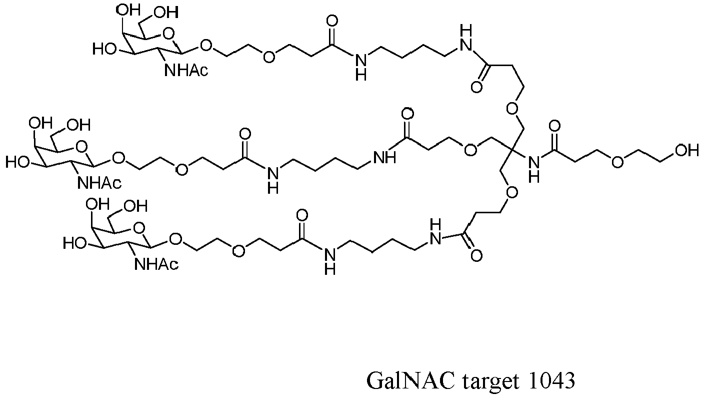

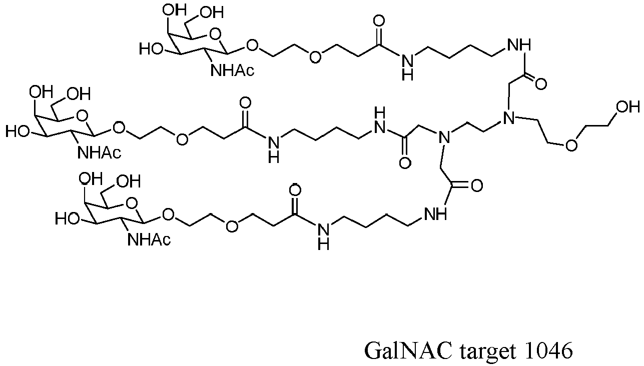

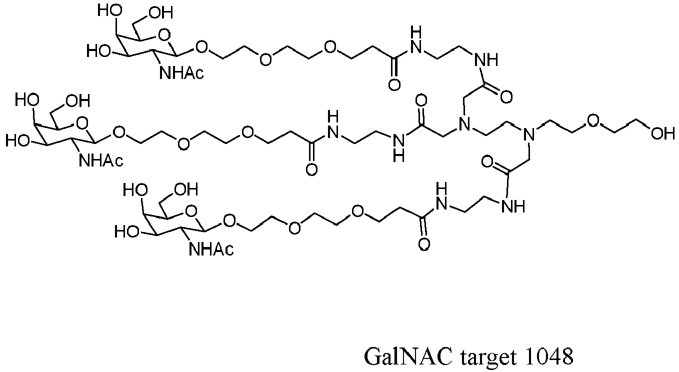

On the other hand, the present invention provides an application of the aforementioned compounds in preparation of a siRNA conjugate.

The compounds of TO23, TO25 and TO26 firstly form intermediates and then covalently link with siRNA, and the intermediates are selected from:

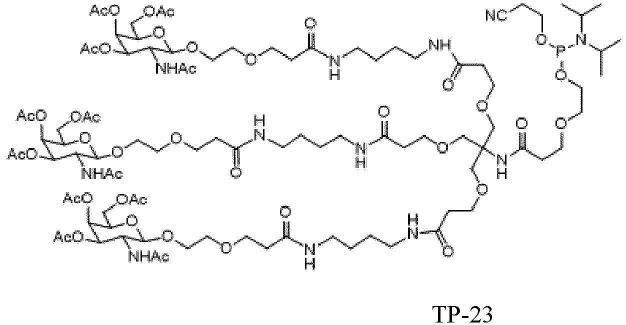

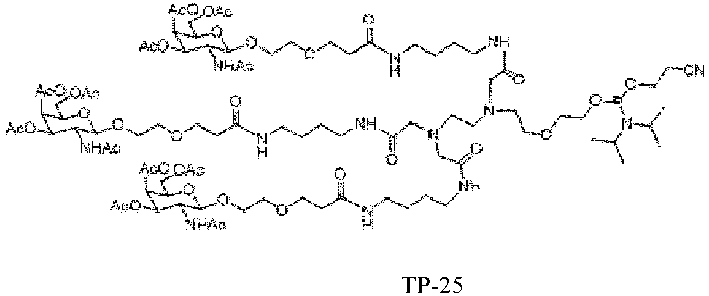

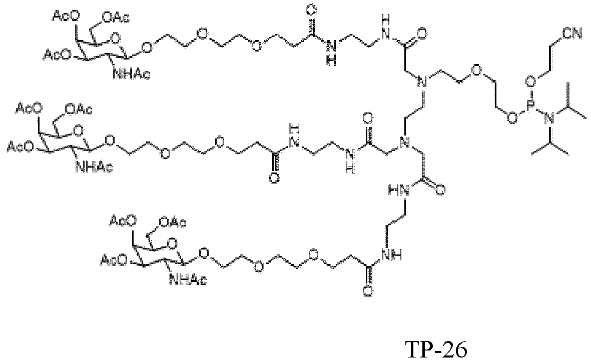

The present disclosure also provides the above intermediates.

On the other hand, the present disclosure provides a use of the aforementioned compounds and/or intermediates in preparation of a drug or kit, and the drug or kit is used to inhibit expression of an *ANGPTL3* gene.

Preferably, the drug or kit is used to prevent and/or treat a dyslipidemia disease; and further preferably, the dyslipidemia disease includes hyperlipidemia and hypertriglyceridemia.

On the other hand, the present disclosure provides a kit. According to an embodiment of the present disclosure, the kit includes the siRNA and/or the siRNA conjugate.

Therefore, the kit according to the embodiment of the present disclosure may be used to inhibit the expression of the *ANGPTL3* gene in the cell, thereby the levels of LDL-C, VLDL-C, HDL-C and TG are reduced, as to prevent and/or treat the hyperlipidemia and the hypertriglyceridemia.

On the other hand, the present disclosure provides a method for inhibiting expression of an *ANGPTL3* gene in a subject, and the method includes: administering the previously described siRNA and/or the previously described siRNA conjugate to the subject, as to inhibit the expression of the *ANGPTL3* gene.

On the other hand, the present disclosure provides a method for inhibiting expression of an *ANGPTL3* gene in a cell. According to an embodiment of the present disclosure, the method includes: transfecting the cell with the siRNA and/or the siRNA conjugate, as to inhibit the expression of the *ANGPTL3* gene in the cell.

According to the method for inhibiting the expression of the *ANGPTL3* gene in the cell in the embodiment of the present disclosure, the siRNA is used to form RISC, and complementary-paired with the mRNA sequence of the target gene (*ANGPTL3* gene) to degrade mRNA of the target gene so as to inhibit the expression of the target gene, and then reduce the levels of LDL-C, VLDL-C, HDL-C and TG

According to an embodiment of the present disclosure, the cell is derived from a mammal.

Optionally, the cell is derived from a human.

Optionally, the cell is a liver cell.

The siRNA provided by the present disclosure is used to form RISC in the human liver cell, and complementary-paired with the mRNA sequence of the *ANGPTL3* gene to degrade mRNA of the *ANGPTL3* gene so as to inhibit its expression, and then reduce the levels of LDL-C, VLDL-C, HDL-C and TG

On the other hand, the present disclosure provides a use of the siRNA and/or the siRNA conjugate in preparation of a drug or a kit. According to an embodiment of the present disclosure, the drug or the kit is used to inhibit the expression of the *ANGPTL3* gene.

The siRNA provided by the present disclosure is used to prepare the drug or the kit, and the drug or the kit reduces the expression level of the *ANGPTL3* gene in the cell by the siRNA therein, thereby the dyslipidemia diseases are prevented and/or treated.

According to an embodiment of the present disclosure, the drug or the kit is used to prevent and/or treat a dyslipidemia disease.

Optionally, the dyslipidemia disease includes the hyperlipidemia and the hypertriglyceridemia.

Optionally, the drug or the kit is used to inhibit the expression of the *ANGPTL3* gene in the cell.

On the other hand, the present disclosure provides a method for preventing and/or treating the dyslipidemia disease. According to an embodiment of the present disclosure, the method includes: administering the siRNA and/or the siRNA conjugate to a subject.

According to an embodiment of the present disclosure, the dyslipidemia disease includes the hyperlipidemia and the hypertriglyceridemia.

Additional aspects and advantages of the present disclosure may be partially given in the following descriptions, and some may become apparent from the following descriptions, or may be understood from the practice of the present disclosure.

The present invention has the following beneficial effects.

After the target ligand provided by the present invention forms the conjugate with siRNA, the conjugate may reduce the expression of ANGPTL3 in both cell model and mouse model, and may reduce the expression quantity by more than 50% compared with a control group, it may be maximally reduced by nearly 90%, and has a good clinical application prospect.

### Brief Description of the Drawings

The above and/or additional aspects and advantages of the present disclosure may become apparent and easily understood from descriptions of embodiments in combination with the following drawings, herein:
Fig. 1 shows an expression result of an *ANGPTL3* gene (abbreviated as ANL3 in the figure) in a Hep 3B cell detected by a quantitative real-time PCR after the cell is transfected by some siRNAs in Table 2 at 0.1 nM concentration.
Fig. 2 shows an expression result of the *ANGPTL3* gene (abbreviated as ANL3 in the figure) in the Hep 3B cell detected by the quantitative real-time PCR after the cell is transfected by some siRNAs in Table 2 at 10 nM concentration.
Fig. 3 shows a GalNAc-siRNA conjugate synthesized in Embodiment 3, herein the structure of the conjugate in the second column includes three portions: Target-Type of Modification-siRNA. For example, the structure of G1043-S2A2-A265 is: the 1043 target is linked with the 5'-end of the siRNA sense chain numbered as A265 by the thiophosphate bond, and S2A2 is the type of modification to siRNA of A265. The specific modification groups and modification modes are as follows.

In the nucleic acid sequence, Ao represents an adenosine, Uo represents a uridine, Go represents a guanosine, and Co represents a cytosine, and there is no symbol between directly adjacent nucleotides, it is indicated that it is linked by a normal phosphate bond.

DNA: AGCT (A represents 2'-deoxyadenosine, T represents 2'-deoxythymidine, G represents 2'-deoxyguanosine, and C represents 2'-deoxycytidine).

2'-F: aFgFcFuF (aF represents 2'-fluoroadenine nucleoside, uF represents 2'-fluorouracil nucleoside, gF represents 2'-fluoroguanine nucleoside, and cFrepresents 2'-fluorocytosine nucleoside).

2'-OMe: aMgMcMuM (aM represents 2'-O-methyladenine nucleoside, uM represents 2'-O-methyluracil nucleoside, gM represents 2'-O-methylguanine nucleoside, and cM represents 2'-O-methylcytosine nucleoside).
*: represents that it is linked by a thiophosphate bond.
y and z in the sequence represent the position of the target.

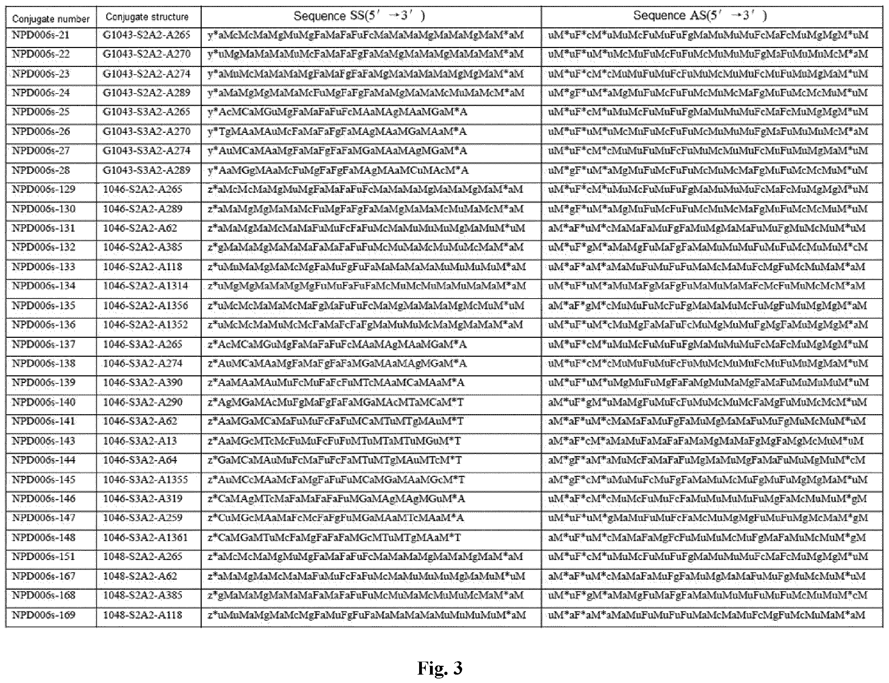

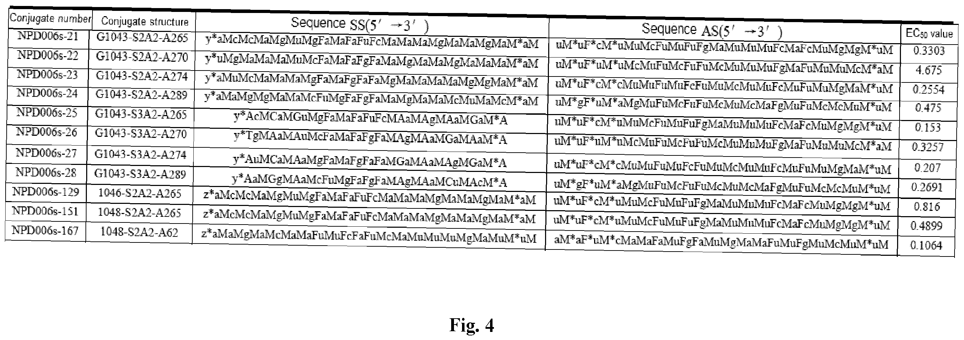

Fig. 4 shows an activity test result (EC₅₀ value) of each conjugate in Embodiment 4.The conjugates in FIG. 4 are included in FIG. 3.

### Detailed Description of the Embodiments

Embodiments of the present disclosure are described in detail below. The embodiments described below are exemplary, and are only used to explain the present disclosure, but may not be understood as limitation to the present disclosure.

"Pharmaceutically acceptable carriers" are recognized in the field, including a pharmaceutically acceptable material, composition or carrier suitable for applying a compound of the present disclosure to a mammal. The carrier includes a liquid or solid filler, a diluent, an excipient, a solvent or an encapsulation material that is involved in carrying or transferring a subject substance from one organ or a part of a body to another organ or another part of the body. Each carrier must be "acceptable" in the sense that it is compatible with other components in a preparation and harmless to a patient. Some examples of materials that may be used as the pharmaceutically acceptable carriers include: sugars, such as a lactose, a glucose, and a sucrose; starches, such as a corn starch and a potato starch; a cellulose and its derivatives, such as a sodium carboxymethyl cellulose, an ethyl cellulose and a cellulose acetate, a powder-like tragacanth gum, a malt, a gelatin, and talcum powder; excipients, such as a cocoa butter and a suppository wax; oils, such as peanut oil, cottonseed oil, safflower oil, sesame oil, olive oil, corn oil and soybean oil; glycols, such as a propylene glycol; polyols, such as a glycerin, a sorbitol, a mannitol and a polyethylene glycol; esters, such as an ethyl oleate and an ethyl laurate; an agar; buffer agents, such as a magnesium hydroxide and an aluminum hydroxide; an alginic acid; pyrogen-free water; Ringer's solution; ethanol; phosphate buffer solution; and other non-toxic compatible substances used in the pharmaceutical preparation.

A wetting agent, an emulsifier and a lubricant such as a sodium dodecyl sulfate and a magnesium stearate, as well as a colorant, a releasing agent, a coating agent, a sweetening agent, a flavoring agent and an aromatic agent, a preservative and an antioxidant may also be present in the composition.

The pharmaceutical composition of the present disclosure includes those suitable for oral, nasal, topical, buccal, sublingual, rectal, and/or parenteral administration. The preparation may conveniently exist in the form of a unit dosage form and may be prepared by any methods well-known in the pharmaceutical field. The amount of an active ingredient that may be combined with the carrier substance to prepare a single dosage form is generally the amount of the compound that produces the therapeutic effect. In general, in the unit of 1%, the amount of the active ingredient is about 1% to about 99%, preferably about 5% to about 70%, and most preferably about 10% to about 30%.

A term "treatment" is used to refer to obtain the desired pharmacological and/or physiological effect. The effect may be preventive in terms of completely or partially preventing a disease or its symptoms, and/or therapeutic in terms of partially or completely curing the disease and/or adverse effects caused by the disease. The "treatment" used herein encompasses diseases of mammals, especially human diseases, including: (a) prevention of diseases or symptoms in individuals who are prone to disease but are not diagnosed with the diseases yet; (b) inhibition of the diseases, such as retardation of disease development; or (c) remission of the diseases, such as the symptoms related to the diseases are alleviated. The "treatment" used herein encompasses any medication that gives a drug or a compound to the individual to treat, cure, remit, improve, alleviate or inhibit the diseases of the individual, including but not limited to giving the drug containing the compound described herein to the individual in need.

The present disclosure provides a siRNA for inhibiting expression of ANGPTL3. According to an embodiment of the present disclosure, the siRNA includes a sense chain and an antisense chain, and the antisense chain includes a complementary region complementary-paired to the sense chain, herein the sense chain is selected from a nucleotide sequence that is not more than 5 nucleotides different from a nucleotide sequence of each chain in SEQ ID NO: 1-SEQ ID NO: 154, and the antisense chain is selected from a nucleotide sequence that is not more than 5 nucleotides different from a nucleotide sequence of each chain in SEQ ID NO: 155-SEQ ID NO: 308.

According to an embodiment of the present disclosure, the sense chain includes not only SEQ ID NO: 1-SEQ ID NO: 154 shown in Table 2, but also a continuous nucleotide sequence that is 1, 2, 3, 4 and 5 nucleotides different from the sense chain shown in Table 2.

According to an embodiment of the present disclosure, the antisense chain includes not only SEQ ID NO: 155-SEQ ID NO: 308 shown in Table 2, but also a continuous nucleotide sequence that is 1, 2, 3, 4 and 5 nucleotides different from the antisense chain shown in Table 2.

According to an embodiment of the present disclosure, the siRNA includes at least one modified nucleotide.

The modified nucleotide is selected from at least one of the following:
a 5'-thiophosphate based nucleotide, a 5-methylcytosine nucleotide, a 2'-O-methyl modified nucleotide, a 2'-O-2-methoxyethyl modified nucleotide, a 2'-fluoro modified nucleotide, a 3'-nitrogen substituted modified nucleotide, a 2'-deoxy-2'-fluoro modified nucleotide, a 2'-deoxy modified nucleotide, a locked nucleotide, a de-base nucleotide, a 2'-amino modified nucleotide, a morpholino nucleotide, a polypeptide nucleotide, an amino phosphate, and a nucleotide including a non natural base.

According to an embodiment of the present disclosure, the length of the complementary region is 18-21 bp, for example, 19 bp.

According to an embodiment of the present disclosure, the lengths of the sense chain and the antisense chain in the siRNA are 18-25 bp, for example, 21 bp.

According to a specific embodiment of the present disclosure, the lengths of the sense chain and the antisense chain in the siRNA are 21 bp, and bases in the sense chain and the antisense chain are complementary one by one, or 19 consecutive bases are complementary in the sense chain and the antisense chain in the siRNA, namely the length of the complementary region is 19 bp.

According to an embodiment of the present disclosure, a liver cell is transfected with the siRNA, as to inhibit the expression of the *ANGPTL3* gene in the cell.

For an *ANGPTL3* gene target, the inventor of the present disclosure designs an appropriate small interfering nucleic acid sequence, synthesizes the siRNA, uses a transfection reagent to introduce the siRNA into the cell, forms RISC, specifically recognizes and targets the mRNA sequence that binds to the target gene, and cuts mRNA between 10-11 bases from a 5'-end, thus the post-transcriptional gene silencing is caused, and the expression of an ANGPTL3 secreted protein is regulated.

According to an embodiment of the present disclosure, the siRNA is linked with a target ligand by a covalent bond.

According to an embodiment of the present disclosure, the target ligand includes at least one N-acetyl-galactosamine.

According to an embodiment of the present disclosure, the target ligand is linked to the sense chain in the siRNA.

The embodiments of the present disclosure are described in detail below. The embodiments described below are exemplary, and are only used to explain the present disclosure, but may not be understood as limitation to the present disclosure. If no specific technologies or conditions are indicated in the embodiments, it is performed according to the technologies or conditions described in documents in this field or product instructions. Reagents or instruments used that do not indicate manufacturers are all conventional products that may be purchased in the market.

Some synthetic routes of this embodiment may refer to CN202110397429.9 and CN202110008013.3; and the embodiments of the present application add the above two patent applications in a mode of source citation.

### Embodiment 1: Activity test of small interfering nucleic acid (siRNA) by in vitro cell model (Hep 3B cell)

1) Preparation of suspension transfection reagent: the concentration of siRNA mother liquor is 50 µM. A diethylpyrocarbonate (DEPC) is diluted with water to obtain 10 µM of a siRNA system, 50 µL of Opti-MEM is diluted to obtain 0.2 µM of the siRNA system, it is blown and sucked for 3-5 times and mixed uniformly (the final concentration is 10 nM). 50 µL of Opti-MEM is diluted with 0.5µL of 0.2µM siRNA to obtain 0.002 µM of the siRNA system, and it is blown and sucked for 3-5 times and mixed uniformly (the final concentration is 0.1 nM); and 50 µL of Opti-MEM is diluted with 2 µL of RNAiMAX, and it is blown and sucked for 3-5 times and mixed uniformly. A transfection reagent and a small interfering nucleic acid diluent are respectively mixed, it is blown and sucked for 3-5 times and mixed uniformly, and stilly placed for 10 min at a room temperature.
2) Cell treatment: it is observed under a microscope that the convergence rate of a Hep 3B cell line is >70%, cells are spread on a 12-well plate according to 2×10⁵ cells/well, 900 µL of a dulbecco's modified eagle medium (DMEM) containing 10% fetal bovine serum (FBS) is added per well, and a transfection complex is added to the 12-well plate, and cultured in a 5% CO2 incubator at 37°C.
3) After 24 h, the total RNA of the cells are extracted, and the expression conditions of the ANGPTL3 mRNA sequence in the cell is detected by the quantitative real-time PCR, herein PCR primers used to amplify internal reference genes peptidylprolylisomerase B (PPIB) and *ANGPTL3* are shown in Table 1.

**Table 1: PCR primer sequence for amplification of internal reference genes PPIB and ANGPTL3**

| Gene name | SEQ ID NO. | Nucleotide sequence (5'-3') |
|---|---|---|
| Human PPIB | 309 | GGTGATCTTTGGTCTCTTCGG |
| | 310 | TAGATGCTCTTTCCTCCTGTG |
| Human ANGPTL3 | 311 | ATTTTAGCCAATGGCCTCCTTC |
| | 312 | CTGGTTTGCAGCGATAGATCATA |

4) The inhibition rate of the small interfering nucleic acid on the expression level of ANGPTL3 is calculated according to the following formula: inhibition rate=[1-(expression quantity of ANGPTL3 mRNA in experimental group/expression quantity of PPIB mRNA in experimental group)/(expression quantity of ANGPTL3 mRNA in negative control group/expression quantity of PPIB mRNA in negative control group)]×100%. Herein, each experimental group is the cells treated with the small interfering nucleic acid respectively; and the negative control group (marked as Blank) is the cells without any small interfering nucleic acid treatment.

The above method is used to obtain the results of the inhibition rate of the *ANGPTL3* gene (NM_014495.4) expression after the Hep 3B cell is transfected by 154 pairs of siRNAs in Table 2 at the concentrations of 0.1 nM and 10 nM respectively.

**Table 2: 154 pairs of siRNA sequences targeting ANGPTL3**

| Name | Position | Sense chain (5'-3') | SEQ ID NO. | Antisense chain (5'-3') | SEQ ID NO. | Inhibition rate (%) | |
|---|---|---|---|---|---|---|---|
| | | | | | | 0.1nM | 10nM |
| A129 | 98-118 | CAGAAUUGAUCA AGACAAUUC | 1 | AUUGUCUUGAUCA AUUCUGGA | 155 | 88 | 78 |
| A554 | 523-543 | CAGAAGUAACUU CACUUAAAA | 2 | UUAAGUGAAGUUA CUUCUGGG | 156 | 78 | 70 |
| A566 | 535-555 | CACUUAAAACUU UUGUAGAAA | 3 | UCUACAAAAGUUU UAAGUGAA | 157 | 44 | 70 |
| A568 | 537-557 | CUUAAAACUUUU GUAGAAAA | 4 | UUUCUACAAAAGU UUUAAGUG | 158 | 58 | 56 |
| A749 | 718-738 | GAACUACUCCCU UUCUUCAGU | 5 | UGAAGAAAGGAG UAGUUCUU | 159 | 82 | 85 |
| A889 | 858-878 | CAUGUCUACUGU GAUGUUAUA | 6 | UAACAUCACAGUA GACAUGAA | 160 | 62 | 78 |
| A1053 | 1022-10 42 | GCAAUCUAAUUA UGUUUUACG | 7 | UAAAACAUAAUUA GAUUGCUU | 161 | 41 | 79 |
| A1058 | 1027-10 47 | CUAAUUAUGUUU UACGAAUUG | 8 | AUUCGUAAAACAU AAUUAGAU | 162 | 52 | 88 |
| A1145 | 1114-11 34 | CCAACUAUACGC UACAUCUAG | 9 | AGAUGUAGCGUAU AGUUGGUU | 163 | 72 | 90 |
| A13 | 60-80 | AAGCUCCUUCUU UUUAUUGUU | 10 | AACAAUAAAAAGA AGGAGCUU | 164 | 84 | 93 |
| A32 | 79-99 | UUCCUCUAGUUA UUUCCUCCA | 11 | UGGAGGAAAUAAC UAGAGGAA | 165 | 64 | 64 |
| A35 | 82-102 | CUCUAGUUAUUU CCUCCAGAA | 12 | UUCUGGAGGAAAU AACUAGAG | 166 | 86 | 90 |
| A45 | 92-112 | UUCCUCCAGAAU UGAUCAAGA | 13 | UCUUGAUCAAUUC UGGAGGAA | 167 | 82 | 87 |
| A48 | 95-115 | CUCCAGAAUUGA UCAAGACAA | 14 | UUGUCUUGAUCAA UUCUGGAG | 168 | 84 | 90 |
| A49 | 96-116 | UCCAGAAUUGAU CAAGACAAU | 15 | AUUGUCUUGAUCA AUUCUGGA | 169 | 79 | 89 |
| A56 | 103-123 | UUGAUCAAGACA AUUCAUCAU | 16 | AUGAUGAAUUGUC UUGAUCAA | 170 | 78 | 88 |
| A62 | 109-129 | AAGACAAUUCAU CAUUUGAUU | 17 | AAUCAAAUGAUGA AUUGUCUU | 171 | 77 | 88 |
| A64 | 111-131 | GACAAUUCAUCA UUUGAUUCU | 18 | AGAAUCAAAUGAU GAAUUGUC | 172 | 69 | 84 |
| A118 | 165-185 | UUAGACGAUGUA AAAAUUUUA | 19 | UAAAAUUUUUACA UCGUCUAA | 173 | 79 | 79 |
| A182 | 229-249 | UCCAUAAGACGA AGGGCCAAA | 20 | UUUGGCCCUUCGU CUUAUGGA | 174 | 23 | 50 |
| A189 | 236-256 | GACGAAGGGCCA AAUUAAUGA | 21 | UCAUUAAUUUGGC CCUUCGUC | 175 | 55 | 57 |
| A206 | 253-273 | AUGACAUAUUUC AAAAACUCA | 22 | UGAGUUUUGAAA UAUGUCAU | 176 | 69 | 82 |
| A207 | 254-274 | UGACAUAUUUCA AAAACUCAA | 23 | UUGAGUUUUUGAA AUAUGUCA | 177 | 75 | 93 |
| A1209 | 1256-12 76 | GUGGCAUGAUGA GUGUGGAGA | 24 | UCUCCACACUCAUC AUGCCAC | 178 | 60 | 87 |
| A236 | 283-303 | AUCAGUCUUUUU AUGAUCUAU | 25 | AUAGAUCAUAAAA AGACUGAU | 179 | 48 | 75 |
| A257 | 304-324 | CGCUGCAAACCA GUGAAAUCA | 26 | UGAUUUCACUGGU UUGCAGCG | 180 | 78 | 87 |
| A259 | 306-326 | CUGCAAACCAGU GAAAUCAAA | 27 | UUUGAUUUCACUG GUUUGCAG | 181 | 84 | 89 |
| A264 | 311-331 | AACCAGUGAAAU CAAAGAAGA | 28 | UCUUCUUUGAUUU CACUGGUU | 182 | 80 | 85 |
| A265 | 312-332 | ACCAGUGAAAUC AAAGAAGAA | 29 | UUCUUCUUUGAUU UCACUGGU | 183 | 66 | 80 |
| A267 | 314-334 | CAGUGAAAUCAA AGAAGAAGA | 30 | UCUUCUUCUUUGA UUUCACUG | 184 | 62 | 76 |
| A270 | 317-337 | UGAAAUCAAAGA AGAAGAAAA | 31 | UUUUCUUCUUCUU UGAUUUCA | 185 | 48 | 86 |
| A274 | 321-341 | AUCAAAGAAGAA GAAAAGGAA | 32 | UUCCUUUUCUUCU UCUUUGAU | 186 | 50 | 85 |
| A281 | 328-348 | AAGAAGAAAAGG AACUGAGAA | 33 | UUCUCAGUUCCUU UUCUUCUU | 187 | 59 | 84 |
| A284 | 331-351 | AAGAAAAGGAAC UGAGAAGAA | 34 | UUCUUCUCAGUUC CUUUCUU | 188 | 47 | 53 |
| A289 | 336-356 | AAGGAACUGAGA AGAACUACA | 35 | UGUAGUUCUUCUC AGUUCCUU | 189 | 77 | 89 |
| A290 | 337-357 | AGGAACUGAGAA GAACUACAU | 36 | AUGUAGUUCUUCU CAGUUCCU | 190 | 60 | 87 |
| A293 | 340-360 | AACUGAGAAGAA CUACAUAUA | 37 | UAUAUGUAGUUCU UCUCAGUU | 191 | 70 | 80 |
| A294 | 341-361 | ACUGAGAAGAAC UACAUAUAA | 38 | UUAUAUGUAGUUC UUCUCAGU | 192 | 43 | 77 |
| A319 | 366-386 | CAAGUCAAAAAU GAAGAGGUA | 39 | UACCUCUUCAUUU UUGACUUG | 193 | 55 | 82 |
| A329 | 376-396 | AUGAAGAGGUAA AGAAUAUGU | 40 | ACAUAUUCUUUAC CUCUUCAU | 194 | 42 | 64 |
| A346 | 393-413 | AUGUCACUUGAA CUCAACUCA | 41 | UGAGUUGAGUUCA AGUGACAU | 195 | 75 | 79 |
| A3 79 | 426-446 | CUCCUAGAAGAA AAAAUUCUA | 42 | UAGAAUUUUUCU UCUAGGAG | 196 | 72 | 77 |
| A3 85 | 432-452 | GAAGAAAAAAUU CUACUUCAA | 43 | UUGAAGUAGAAUU UUUUCUUC | 197 | 63 | 80 |
| A390 | 437-457 | AAAAAUUCUACU UCAACAAAA | 44 | UUUUGUUGAAGUA GAAUUUU | 198 | 73 | 81 |
| A391 | 438-458 | AAAAUUCUACUU CAACAAAA | 45 | UUUUUGUUGAAGU AGAAUUUU | 199 | 69 | 73 |
| A397 | 444-464 | CUACUUCAACAA AAAGUGAAA | 46 | UUUCACUUUUUGU UGAAGUAG | 200 | 52 | 67 |
| A398 | 445-465 | UACUUCAACAAA AAGUGAAAU | 47 | AUUUCACUUUUUG UUGAAGUA | 201 | 22 | 28 |
| A401 | 448-468 | UUCAACAAAAAG UGAAAUAUU | 48 | AAUAUUUCACUUU UUGUUGAA | 202 | 56 | 66 |
| A403 | 450-470 | CAACAAAAAGUG AAAUAUUUA | 49 | UAAAUAUUUCACU UUUUGUUG | 203 | 47 | 64 |
| A462 | 509-529 | AACUCCAGAACA CCCAGAAGU | 50 | ACUUCUGGGUGUU CUGGAGUU | 204 | 64 | 74 |
| A464 | 511-531 | CUCCAGAACACCC AGAAGUAA | 51 | UUACUUCUGGGUG UUCUGGAG | 205 | 51 | 81 |
| A473 | 520-540 | ACCCAGAAGUAA CUUCACUUA | 52 | UAAGUGAAGUUAC UUCUGGGU | 206 | 56 | 71 |
| A475 | 522-542 | CCAGAAGUAACU UCACUUAAA | 53 | UUUAAGUGAAGUU ACUUCUGG | 207 | 65 | 68 |
| A476 | 523-543 | CAGAAGUAACUU CACUUAAAA | 54 | UUUUAAGUGAAGU UACUUCUG | 208 | 65 | 71 |
| A479 | 526-546 | AAGUAACUUCAC UUAAAACUU | 55 | AAGUUUUAAGUGA AGUUACUU | 209 | 21 | 78 |
| A483 | 530-550 | AACUUCACUUAA AACUUUGU | 56 | ACAAAAGUUUUAA GUGAAGUU | 210 | 39 | 34 |
| A495 | 542-562 | AACUUUUGUAGA AAAACAAGA | 57 | UCUUGUUUUCUA CAAAAGUU | 211 | 55 | 76 |
| A500 | 547-567 | UUGUAGAAAAC AAGAUAAUA | 58 | UAUUAUCUUGUUU UUCUACAA | 212 | 52 | 54 |
| A508 | 555-575 | AAACAAGAUAAU AGCAUCAAA | 59 | UUUGAUGCUAUUA UCUUGUUU | 213 | 50 | 74 |
| A519 | 566-586 | UAGCAUCAAAGA CCUUCUCCA | 60 | UGGAGAAGGUCUU UGAUGCUA | 214 | 69 | 77 |
| A537 | 584-604 | CCAGACCGUGGA AGACCAAUA | 61 | UAUUGGUCUUCCA CGGUCUGG | 215 | 64 | 25 |
| A538 | 585-605 | CAGACCGUGGAA GACCAAUAU | 62 | AUAUUGGUCUUCC ACGGUCUG | 216 | 43 | - |
| A540 | 587-607 | GACCGUGGAAGA CCAAUAUAA | 63 | UUAUAUUGGUCUU CCACGGUC | 217 | 37 | 58 |
| A541 | 588-608 | ACCGUGGAAGAC CAAUAUAAA | 64 | UUUAUAUUGGUCU UCCACGGU | 218 | 40 | 59 |
| A544 | 591-611 | GUGGAAGACCAA UAUAAACAA | 65 | UUGUUUAUAUUGG UCUUCCAC | 219 | Invalid | 38 |
| A547 | 594-614 | GAAGACCAAUAU AAACAAUUA | 66 | UAAUUGUUUAUAU UGGUCUUC | 220 | 36 | 60 |
| A548 | 595-615 | AAGACCAAUAUA AACAAUUAA | 67 | UUAAUUGUUUAUA UUGGUCUU | 221 | 11 | 13 |
| A568 | 615-635 | AACCAACAGCAU AGUCAAAUA | 68 | UAUUUGACUAUGC UGUUGGUU | 222 | 24 | 58 |
| A569 | 616-636 | ACCAACAGCAUA GUCAAAUAA | 69 | UUAUUUGACUAUG CUGUUGGU | 223 | 17 | 36 |
| A579 | 626-646 | UAGUCAAAUAAA AGAAAUAGA | 70 | UCUAUUUCUUUUA UUUGACUA | 224 | 29 | 72 |
| A582 | 629-649 | UCAAAUAAAAGA AAUAGAAAA | 71 | UUUUCUAUUUCUU UUAUUUGA | 225 | 22 | 44 |
| A602 | 649-669 | AUCAGCUCAGAA GGACUAGUA | 72 | UACUAGUCCUUCU GAGCUGAU | 226 | 47 | 75 |
| A604 | 651-671 | CAGCUCAGAAGG ACUAGUAUU | 73 | AAUACUAGUCCUU CUGAGCUG | 227 | 44 | 69 |
| A607 | 654-674 | CUCAGAAGGACU AGUAUUCAA | 74 | UUGAAUACUAGUC CUUCUGAG | 228 | 36 | 67 |
| A609 | 656-676 | CAGAAGGACUAG UAUUCAAGA | 75 | UCUUGAAUACUAG UCCUUCUG | 229 | 21 | 49 |
| A618 | 665-685 | UAGUAUUCAAGA ACCCACAGA | 76 | UCUGUGGGUUCUU GAAUACUA | 230 | 16 | 61 |
| A629 | 676-696 | AACCCACAGAAA UUUCUCUAU | 77 | AUAGAGAAAUUUC UGUGGGUU | 231 | 29 | 38 |
| A652 | 699-719 | UCCAAGCCAAGA GCACCAAGA | 78 | UCUUGGUGCUCUU GGCUUGGA | 232 | 40 | 78 |
| A655 | 702-722 | AAGCCAAGAGCA CCAAGAACU | 79 | AGUUCUUGGUGCU CUUGGCUU | 233 | 44 | 70 |
| A675 | 722-745 | UACUCCCUUUCU UCAGUUGAA | 80 | UUCAACUGAAGAA AGGGAGUA | 234 | 50 | 72 |
| A678 | 725-745 | UCCCUUUCUUCA GUUGAAUGA | 81 | UCAUUCAACUGAA GAAAGGA | 235 | 57 | 73 |
| A686 | 733-753 | UUCAGUUGAAUG AAAUAAGAA | 82 | UUCUUAUUUCAUU CAACUGAA | 236 | 36 | 55 |
| A687 | 734-754 | UCAGUUGAAUGA AAUAAGAAA | 83 | UUUCUUAUUUCAU UCAACUGA | 237 | 34 | 74 |
| A691 | 738-758 | UUGAAUGAAAUA AGAAAUGUA | 84 | UACAUUUCUUAUU UCAUUCAA | 238 | 52 | 72 |
| A725 | 772-792 | UUCCUGCUGAAU GUACCACCA | 85 | UGGUGGUACAUUC AGCAGGAA | 239 | 21 | 60 |
| A729 | 776-796 | UGCUGAAUGUAC CACCAUUUA | 86 | UAAAUGGUGGUAC AUUCAGCA | 240 | 22 | 51 |
| A731 | 778-798 | CUGAAUGUACCA CCAUUUAUA | 87 | UAUAAAUGGUGGU ACAUUCAG | 241 | 58 | 77 |
| A739 | 786-806 | ACCACCAUUUAU AACAGAGGU | 88 | ACCUCUGUUAUAA AUGGUGGU | 242 | 22 | 35 |
| A741 | 788-808 | CACCAUUUAUAA CAGAGGUGA | 89 | UCACCUCUGUUAU AAAUGGUG | 243 | 46 | 74 |
| A742 | 789-809 | ACCAUUUAUAAC AGAGGUGAA | 90 | UUCACCUCUGUUA UAAAUGGU | 244 | 23 | 72 |
| A751 | 798-818 | AACAGAGGUGAA CAUACAAGU | 91 | ACUUGUAUGUUCA CCUCUGUU | 245 | 21 | 68 |
| A755 | 802-822 | GAGGUGAACAUA CAAGUGGCA | 92 | UGCCACUUGUAUG UUCACCUC | 246 | Invalid | 59 |
| A75 8 | 805-825 | GUGAACAUACAA GUGGCAUGU | 93 | ACAUGCCACUUGU AUGUUCAC | 247 | 15 | 55 |
| A765 | 812-832 | UACAAGUGGCAU GUAUGCCAU | 94 | AUGGCAUACAUGC CACUUGUA | 248 | 2 | Invalid |
| A798 | 845-865 | CUCUCAAGUUUU UCAUGUCUA | 95 | UAGACAUGAAAAA CUUGAGAG | 249 | 40 | 64 |
| A809 | 856-876 | UUCAUGUCUACU GUGAUGUUA | 96 | UAACAUCACAGUA GACAUGAA | 250 | 66 | 69 |
| A811 | 858-878 | CAUGUCUACUGU GAUGUUAUA | 97 | UAUAACAUCACAG UAGACAUG | 251 | 30 | 54 |
| A814 | 861-881 | GUCUACUGUGAU GUUAUAUCA | 98 | UGAUAUAACAUCA CAGUAGAC | 252 | 70 | 74 |
| A817 | 864-884 | UACUGUGAUGUU AUAUCAGGU | 99 | ACCUGAUAUAACA UCACAGUA | 253 | 65 | 63 |
| A833 | 880-900 | CAGGUAGUCCAU GGACAUUAA | 100 | UUAAUGUCCAUGG ACUACCUG | 254 | 34 | 36 |
| A854 | 901-921 | UUCAACAUCGAA UAGAUGGAU | 101 | AUCCAUCUAUUCG AUGUUGAA | 255 | 27 | 44 |
| A866 | 913-933 | UAGAUGGAUCAC AAAACUUCA | 102 | UGAAGUUUUGUGA UCCAUCUA | 256 | 71 | 83 |
| A870 | 917-937 | UGGAUCACAAAA CUUCAAUGA | 103 | UCAUUGAAGUUUU GUGAUCCA | 257 | 75 | 79 |
| A875 | 922-942 | CACAAAACUUCA AUGAAACGU | 104 | ACGUUUCAUUGAA GUUUGUG | 258 | 68 | 78 |
| A887 | 934-954 | AUGAAACGUGGG AGAACUACA | 105 | UGUAGUUCUCCCA CGUUUCAU | 259 | 74 | 76 |
| A891 | 938-958 | AACGUGGGAGAA CUACAAAUA | 106 | UAUUUGUAGUUCU CCCACGUU | 260 | 36 | 48 |
| A898 | 945-965 | GAGAACUACAAA UAUGGUUUU | 107 | AAAACCAUAUUUG UAGUUCUC | 261 | 63 | 73 |
| A1004 | 1051-10 71 | UGGAAGACUGGA AAGACAACA | 108 | UGUUGUCUUUCCA GUCUUCCA | 262 | 43 | 76 |
| A1006 | 1053-10 73 | GAAGACUGGAAA GACAACAAA | 109 | UUUGUUGUCUUUC CAGUCUUC | 263 | 70 | 79 |
| A1011 | 1058-10 78 | CUGGAAAGACAA CAAACAUUA | 110 | UAAUGUUUGUUGU CUUUCCAG | 264 | 72 | 77 |
| A1012 | 1059-10 79 | UGGAAAGACAAC AAACAUUAU | 111 | AUAAUGUUUGUUG UCUUUCCA | 265 | 60 | 74 |
| A1018 | 1065-10 85 | GACAACAAACAU UAUAUUGAA | 112 | UUCAAUAUAAUGU UUGUUGUC | 266 | 57 | 85 |
| A1021 | 1068-10 88 | AACAAACAUUAU AUUGAAUAU | 113 | AUAUUCAAUAUAA UGUUUGUU | 267 | 41 | 25 |
| A1025 | 1072-10 92 | AACAUUAUAUUG AAUAUUCUU | 114 | AAGAAUAUUCAAU AUAAUGUU | 268 | 38 | 62 |
| A1066 | 1113-11 33 | ACCAACUAUACG CUACAUCUA | 115 | UAGAUGUAGCGUA UAGUUGGU | 269 | 75 | 84 |
| A1074 | 1121-11 41 | UACGCUACAUCU AGUUGCGAU | 116 | AUCGCAACUAGAU GUAGCGUA | 270 | 69 | 83 |
| A1075 | 1122-11 42 | ACGCUACAUCUA GUUGCGAUU | 117 | AAUCGCAACUAGA UGUAGCGU | 271 | 72 | 80 |
| A1082 | 1129-11 49 | AUCUAGUUGCGA UUACUGGCA | 118 | UGCCAGUAAUCGC AACUAGAU | 272 | 45 | 25 |
| A1097 | 1144-11 64 | CUGGCAAUGUCC CCAAUGCAA | 119 | UUGCAUUGGGGAC AUUGCCAG | 273 | 59 | 62 |
| A1106 | 1153-11 73 | UCCCCAAUGCAA UCCCGGAAA | 120 | UUUCCGGGAUUGC AUUGGGGA | 274 | Invalid | 13 |
| A1107 | 1154-11 74 | CCCCAAUGCAAU CCCGGAAAA | 121 | UUUUCCGGGAUUG CAUUGGGG | 275 | 45 | 54 |
| A1119 | 1166-11 86 | CCCGGAAAACAA AGAUUUGGU | 122 | ACCAAAUCUUUGU UUUCCGGG | 276 | 6 | 43 |
| A1158 | 1205-12 25 | CAAAGCAAAAGG ACACUUCAA | 123 | UUGAAGUGUCCUU UUGCUUUG | 277 | 23 | 73 |
| A1160 | 1207-12 27 | AAGCAAAAGGAC ACUUCAACU | 124 | AGUUGAAGUGUCC UUUGCUU | 278 | 46 | 76 |
| A1167 | 1214-12 34 | AGGACACUUCAA CUGUCCAGA | 125 | UCUGGACAGUUGA AGUGUCCU | 279 | 26 | 48 |
| A1171 | 1218-12 38 | CACUUCAACUGU CCAGAGGGU | 126 | ACCCUCUGGACAG UUGAAGUG | 280 | 48 | 74 |
| A1174 | 1221-12 41 | UUCAACUGUCCA GAGGGUUAU | 127 | AUAACCCUCUGGA CAGUUGAA | 281 | 54 | 79 |
| A1184 | 1231-12 51 | CAGAGGGUUAUU CAGGAGGCU | 128 | AGCCUCCUGAAUA ACCCUCUG | 282 | 33 | 27 |
| A1204 | 1251-12 71 | UGGUGGUGGCAU GAUGAGUGU | 129 | ACACUCAUCAUGC CACCACCA | 283 | 39 | 58 |
| A1207 | 1254-12 74 | UGGUGGCAUGAU GAGUGUGGA | 130 | UCCACACUCAUCA UGCCACCA | 284 | 44 | 74 |
| A1210 | 1257-12 77 | UGGCAUGAUGAG UGUGGAGAA | 131 | UUCUCCACACUCA UCAUGCCA | 285 | 52 | 78 |
| A1211 | 1258-12 78 | GGCAUGAUGAGU GUGGAGAAA | 132 | UUUCUCCACACUC AUCAUGCC | 286 | 44 | 74 |
| A1241 | 1288-13 08 | AUGGUAAAUAUA ACAAACCAA | 133 | UUGGUUUGUUAUA UUUACCAU | 287 | 32 | 81 |
| A1253 | 1300-13 20 | ACAAACCAAGAG CAAAAUCUA | 134 | UAGAUUUUGCUCU UGGUUUGU | 288 | 74 | 82 |
| A1254 | 1301-13 21 | CAAACCAAGAGC AAAAUCUAA | 135 | UUAGAUUUUGCUC UUGGUUUG | 289 | 53 | 84 |
| A1274 | 1321-13 41 | AGCCAGAGAGGA GAAGAGGAU | 136 | AUCCUCUUCUCCUC UCUGGCU | 290 | 39 | 70 |
| A1276 | 1323-13 43 | CCAGAGAGGAGA AGAGGAUUA | 137 | UAAUCCUCUUCUC CUCUCUGG | 291 | 52 | 68 |
| A1277 | 1324-13 44 | CAGAGAGGAGAA GAGGAUUAU | 138 | AUAAUCCUCUUCU CCUCUCUG | 292 | 50 | 73 |
| A1279 | 1326-13 46 | GAGAGGAGAAGA GGAUUAUCU | 139 | AGAUAAUCCUCUU CUCCUCUC | 293 | 67 | 68 |
| A1284 | 1331-13 51 | GAGAAGAGGAUU AUCUUGGAA | 140 | UUCCAAGAUAAUC CUCUUCUC | 294 | 75 | 35 |
| A1303 | 1350-13 70 | AAGUCUCAAAAU GGAAGGUUA | 141 | UAACCUUCCAUUU UGAGACUU | 295 | 64 | 74 |
| A1305 | 1352-13 72 | GUCUCAAAAUGG AAGGUUAUA | 142 | UAUAACCUUCCAU UUUGAGAC | 296 | 51 | 75 |
| A1310 | 1357-13 77 | AAAAUGGAAGGU UAUACUCUA | 143 | UAGAGUAUAACCU UCCAUUUU | 297 | 61 | 71 |
| A1313 | 1360-13 80 | AUGGAAGGUUAU ACUCUAUAA | 144 | UUAUAGAGUAUAA CCUUCCAU | 298 | 33 | 71 |
| A1314 | 1361-13 81 | UGGAAGGUUAUA CUCUAUAAA | 145 | UUUAUAGAGUAUA ACCUUCCA | 299 | 58 | 79 |
| A1318 | 1365-13 85 | AGGUUAUACUCU AUAAAAUCA | 146 | UGAUUUUAUAGAG UAUAACCU | 300 | 48 | 71 |
| A1345 | 1392-14 12 | AUGUUGAUCCAU CCAACAGAU | 147 | AUCUGUUGGAUGG AUCAACAU | 301 | 63 | 80 |
| A1348 | 1395-14 15 | UUGAUCCAUCCA ACAGAUUCA | 148 | UGAAUCUGUUGGA UGGAUCAA | 302 | 58 | 83 |
| A1351 | 1398-14 18 | AUCCAUCCAACA GAUUCAGAA | 149 | UUCUGAAUCUGUU GGAUGGAU | 303 | 61 | 72 |
| A1352 | 1399-14 19 | UCCAUCCAACAG AUUCAGAAA | 150 | UUUCUGAAUCUGU UGGAUGGA | 304 | 60 | 84 |
| A1355 | 1402-14 22 | AUCCAACAGAUU CAGAAAGCU | 151 | AGCUUUCUGAAUC UGUUGGAU | 305 | 73 | 82 |
| A1356 | 1403-14 23 | UCCAACAGAUUC AGAAAGCUU | 152 | AAGCUUUCUGAAU CUGUUGGA | 306 | 53 | 79 |
| A1359 | 1406-14 26 | AACAGAUUCAGA AAGCUUUGA | 153 | UCAAAGCUUUCUG AAUCUGUU | 307 | 62 | 79 |
| A1361 | 1408-14 28 | CAGAUUCAGAAA GCUUUGAAU | 154 | AUUCAAAGCUUUC UGAAUCUG | 308 | 77 | 88 |

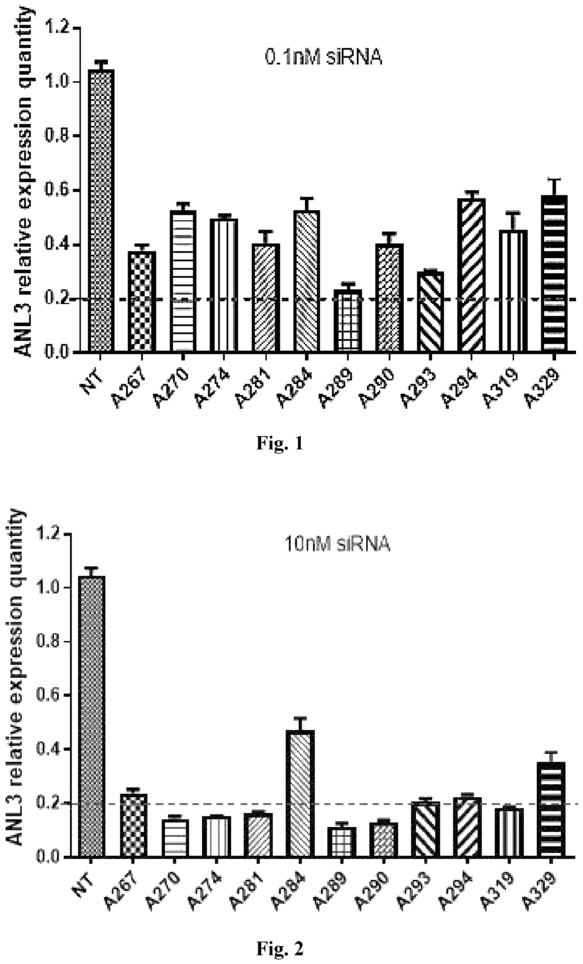

Figs. 1 and 2 respectively show the results of the expression quantity of the *ANGPTL3* gene in the Hep3B cell detected by the quantitative real-time PCR after the cell is transfected by some siRNAs in Table 2 at the concentration of 0.1 nM or 10 nM. It is indicated that the siRNA shown in the drawings may significantly reduce the expression of the *ANGPTL3* gene whether the Hep 3B cell is transfected by the siRNA at the 0.1 nM or 10 nM concentration.

### Embodiment 2: Synthesis of GalNAc linkage target

### I. Synthesis of GalNAc target 1043

According to the following method, a diastereoisomer of TO-23 and TP-23 (a precursor of a 1043 target linked to siRNA) is synthesized.

### 1. Synthesis of intermediate GN-17-01

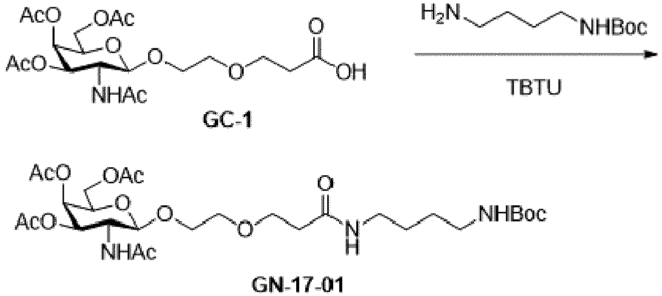

(1) Under an N₂ atmosphere, GC-1 (12 g, 25.89 mmol) is dissolved in a dichloromethane (DCM) (200 mL), the temperature is reduced to 0-5°C in an ice-water bath, O-benzotriazole-N,N,N',N'-tetramethyl-uronium-hexafluorophosphate (HBTU) (11.78 g, 31 mmol) and diisopropylethylamine (DIEA) (10 g, 77.67 mmol) are added, and stirred for 10 minutes.
(2) Then, N-tert-butyloxycarbonyl-1,4-butanediamine (4.87 g, 25.89 mmol) is added, the temperature is risen to 25°C and it is stirred and reacted for 16 hours. A thin-layer chromatography (TLC) shows that raw materials are basically disappeared.
(3) Saturated ammonium chloride solution (100 mL) is added for quenching, solution is separated, and it is extracted by DCM (100 mL×2).
(4) Organic phases are combined and washed with saturated salt water (100 mL), dried with anhydrous Na₂SO₄, filtered and concentrated. After column chromatography purification (DCM/MeOH=20/1), a white solid compound GN-17-01 (15 g, yield: 91%) is obtained.

### 2. Synthesis of intermediate GN-17

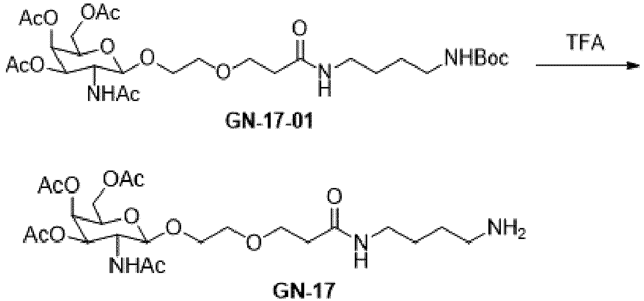

(1) GN-17-01 (15 g, 23.67 mmol) is dissolved in DCM (150 mL), a trifluoroacetic acid (TFA) (50 mL) is added, and stirred at 25°C for 1 hour. TLC shows that raw materials are basically disappeared and concentrated.
(2) The excess TFA is removed by an acetonitrile (100 mL×3) azeotropic with TFA, to obtain a foam-like solid GN-17 (TFA salt, 12.6 g).

### 3. Synthesis of intermediate TO-23-01

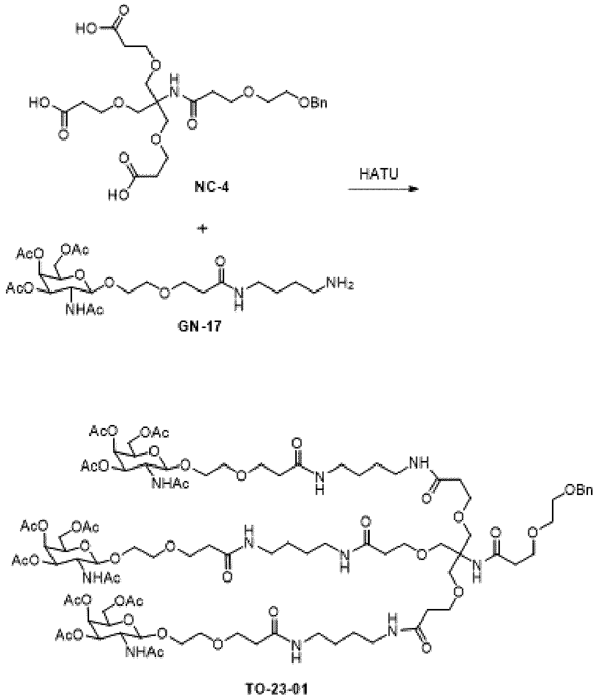

(1) Under the N₂ atmosphere, NC-4 (2.6 g, 4.7 mmol) is dissolved in DCM (200 mL), the temperature is reduced to 0-5°C in the ice-water bath, HATU (5.6 g, 14.83 mmol) and DIEA (4.85 g, 37.6 mmol) are added and stirred for 20 minutes.
(2) Then, GN-17 (8.45 g, 15.5 mmol) is added, the temperature is risen to 25°C and it is stirred and reacted for 4 hours. TLC detection shows that raw materials are basically disappeared.
(3) The saturated ammonium chloride solution (50 mL) is added for quenching, solution is separated, and it is extracted by DCM (100 mL×2).
(4) Organic phases are combined and washed with the saturated salt water (100 mL), and dried with the anhydrous Na₂SO₄.
(5) It is filtered and concentrated to obtain a crude product. After the column chromatography purification (DCM/MeOH=10/1), a white solid TO-23-01 (6.3 g, yield: 63.1%) is obtained.

### 4. Synthesis of compound TO-23

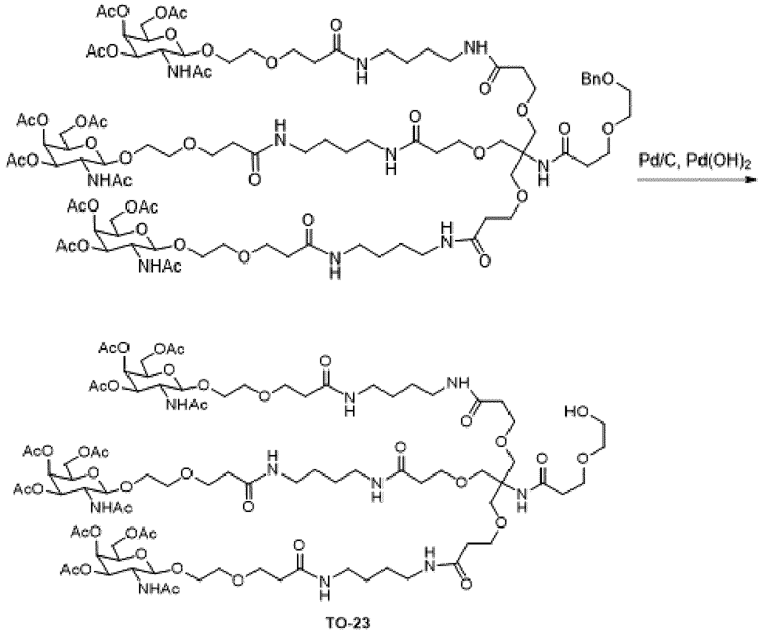

(1) 10% Pd/C (600 mg) and Pd (OH)₂/C (600 mg) are added to MeOH (100 mL) solution of TO-23-01 (6.3 g, 3.0 mmol), it is replaced with H₂ for 3 times, and it is stirred and reacted at 25°C for 3 hours. It is detected by TLC (DCM/MeOH=8/1) that raw materials are basically disappeared.
(2) It is filtered and concentrated to obtain a crude product. After the column chromatography purification (DCM/MeOH/TEA=10/1/0.1), a white solid TO-23 (4.5 g, yield: 75%) is obtained.

¹H NMR (400 MHz, DMSO-*d*₆) δ 7.88-7.81 (m, 9H), 7.14 (s, 1H), 5.21 (d, *J* = 3.4 Hz, 3H), 4.95 (dd, *J* = 11.2, 3.4 Hz, 3H), 4.53 (d, *J* = 8.5 Hz, 3H), 4.07-3.97 (m, 9H), 3.88 (dt, *J* = 11.0, 9.0 Hz, 3H), 3.77-3.71 (m, 3H), 3.63-3.50 (m, 24H), 3.49-3.41 (m, 8H), 3.38-3.35 (m, 2H), 3.08-2.98 (m, 12H), 2.35- 2.25 (m, 14H), 2.10 (s, 9H), 2.00 (s, 9H), 1.89 (s, 9H), 1.78 (s, 9H), 1.40-1.33 (s, 12H).

MS (ESI): m/z [1/2M+H]+theoretical value 1000.5, measured value 1000.3.

### 5. Synthesis of compound TP-23 (precursor of 1043 target linked to siRNA)

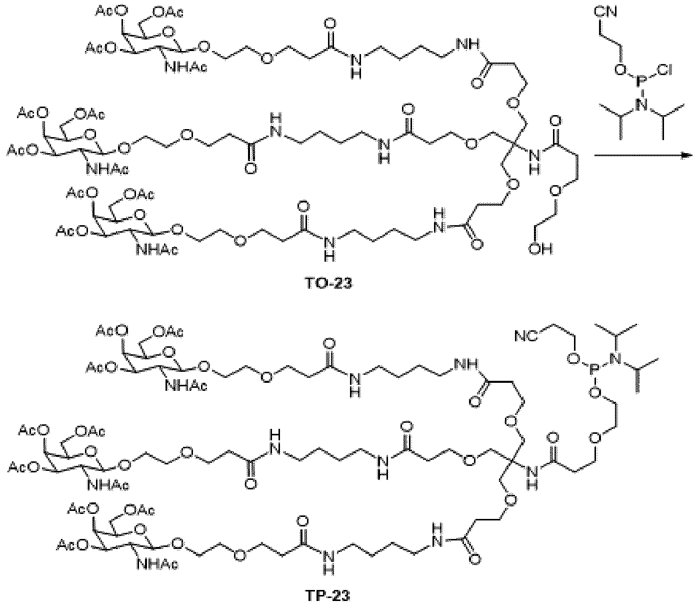

(1) Under the N₂ atmosphere, TO-23 (2.3 g, 1.15 mmol) is dissolved in dry DCM (40 mL), DIEA (0.86 mL, 5.2 mmol) is added, and dry DCM (2 mL) solution of 2-cyanoethyl-N, N-diisopropylchlorophosphoramidite (0.46 mL, 2.1 mmol) is slowly dripped with an injector. It is reacted at 25°C for 1 hour. It is detected by TLC that raw materials are basically disappeared.
(2) Saturated NaHCO₃ (20 mL) is added for quenching, solution is separated, an organic phase is washed with saturated NaHCO₃ (20 mL) solution and saturated salt water (20 mL), dried with the anhydrous MgSO₄, filtered and concentrated to obtain a crude product. After the column chromatography purification (a silica gel column is alkalized by 1.5% TEA/DCM in advance, DCM/MeOH/TEA=15/1/0.1), a white solid TP-23 (1.8 g, yield: 71.1%) is obtained.

¹H NMR (400 MHz, DMSO-*d*₆) δ 7.91-7.79 (m, 9H), 7.15 (s, 1H), 5.21 (d, *J* = 3.4 Hz, 3H), 4.95 (dd, *J* = 11.2, 3.4 Hz, 3H), 4.53 (d, *J* = 8.5 Hz, 3H), 4.06-3.97 (m, 9H), 3.88 (dt, *J* = 11.1, 8.9 Hz, 3H), 3.78-3.66 (m, 6H), 3.63-3.41 (m, 36H), 3.07-2.98 (m, 12H), 2.76 (t, *J* = 5.9 Hz, 2H), 2.35-2.24 (m, 14H), 2.10 (s, 9H), 2.00 (s, 9H), 1.89 (s, 9H), 1.78 (s, 9H), 1.40-1.33 (m, 12H), 1.13 (dd, *J* = 6.7, 4.1 Hz, 12H);

³¹P NMR (162 MHz, DMSO-*d*₆) δ 147.81; and

MS (ESI): m/z[1/2M+Na]+theoretical value 1122.5, measured value 1122.4.

### II. Synthesis of GalNAc target 1046

According to the following method, a diastereoisomer of TO25 and TP-25 (a precursor of a 1046 target linked to siRNA) is synthesized.

### 1. Synthesis of intermediate NC-6-01

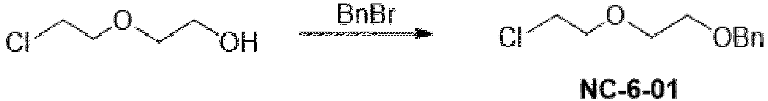

(1) Under the N₂ atmosphere, a dry tetrahydrofuran (THF) (300 mL) is added to a 1000 mL three-necked bottle, the temperature is reduced to 0-5°C in an ice bath and it is stirred, 60% NaH (14 g, 354.8 mmol) is added in batches, then THF solution (200 mL) of 2-chloroethoxyethanol (40 g, 322.5 mmol) is slowly dripped, the temperature is kept and it is reacted for 30 minutes, then a benzyl bromide (60.3 g, 354.8 mmol) is dropwise added to a reaction bottle, the temperature is risen to 25°C and it is stirred for 16 hours. It is monitored by TLC that raw materials are basically consumed.
(2) The saturated ammonium chloride solution (150 mL) is slowly dripped for quenching, solution is separated, a aqueous phase is extracted with an ethyl acetate (EtOAc) (100 mL×2), organic phases are combined and washed with the saturated salt water (300 mL), dried with the anhydrous Na₂SO₄, filtered and concentrated to obtain a crude product. The crude product is purified by a silica gel column chromatography (petroleum ether/EtOAc=5/1) to obtain a yellowish oil-like compound NC-6-01 (53 g, yield: 78%).

MS (ESI): m/z [M+H]+theoretical value 215.1, measured value 215.1.

### 2. Synthesis of intermediate NC-6-02

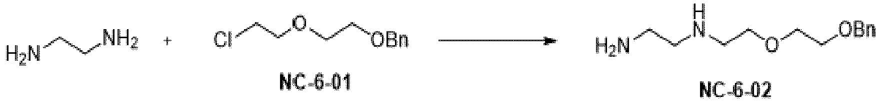

(1) An ethylenediamine (196 g, 3.26 mol) is placed in a 2000 mL three-necked bottle, an acetonitrile (1000 mL), a potassium carbonate (90 g, 0.65 mol) and a sodium iodide (60.6 g, 0.33 mol) are added and stirred. Then, acetonitrile (100 mL) solution of NC-6-01 (70 g, 0.33 mol) is slowly dripped into a reaction bottle, the temperature is risen to 60°C and it is stirred for 16 hours. It is detected by TLC that raw materials are basically consumed.
(2) A reaction is stopped, it is concentrated, purified water (300 mL) is added, pH is adjusted to 4-5 with a concentrated hydrochloric acid, it is extracted for three times with EtOAc (200 mL×3), a sodium hydroxide solid is added into a aqueous phase so that pH is adjusted to 13-14, it is extracted for three times by DCM (200 mL×3), organic phases are combined and washed with the saturated salt water (300 mL), dried with the anhydrous Na₂SO₄, filtered and concentrated to obtain a yellowish oil-like substance NC-6-02 (69.5 g, 87%).

MS (ESI): m/z [M+H]+theoretical value 239.2, measured value 239.1.

### 3. Synthesis of intermediate NC-6-03

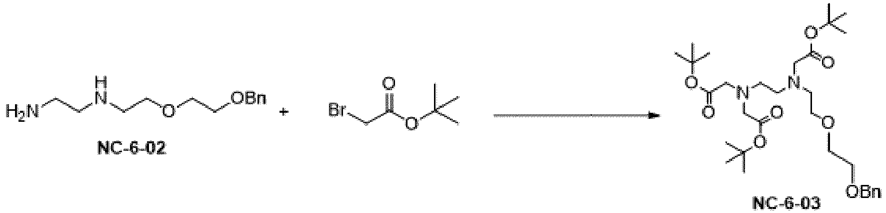

(1) NC-6-02 (69.5 g, 0.29 mol) and tert-butyl bromoacetate (187 g, 0.96 mol) are added to THF (700 mL) and purified water (350 mL), it is stirred, the temperature is reduced below 5°C in the ice-water bath, and a potassium carbonate (322 g, 2.34 mol) is added. It is stirred and reacted at 25°C for 14 hours. It is detected by TLC that raw materials are completely converted.
(2) The purified water (300 mL) is added to reaction solution, it is stilly placed and layered, organic phases are separated, a aqueous phase is extracted for two times with EtOAc (200 mL×2), the organic phases are combined, the saturated salt water (500 mL) is added for washing, and it is dried with the anhydrous Na₂SO₄, filtered and concentrated to obtain a yellowish oil-like substance NC-6-03 (201 g).

MS (ESI): m/z [M+H]+theoretical value 581.4, measured value 581.3.

### 4. Synthesis of intermediate NC-6

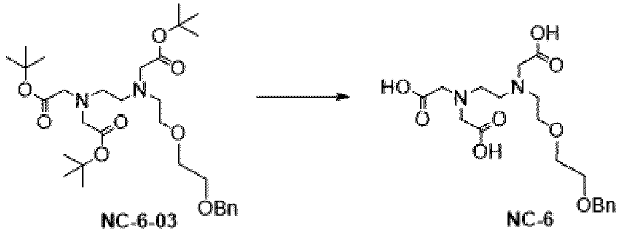

(1) NC-6-03 (23 g, 39.6 mmol) is dissolved in 1,4-dioxane (200 mL), a concentrated hydrochloric acid (40 mL) is added, the temperature is risen to 60°C and it is reacted for 2 hours. It is detected by TLC that raw materials are basically consumed.
(2) It is concentrated, 1,4-dioxane (200 mL) is added again for concentration, to obtain a white solid crude product. The crude product is added to EtOAc (200 mL), it is pulped for 2 hours, suction-filtered to collect a filter cake, and vacuum-dried at 50°C to obtain a white solid compound NC-6 (22.6 g, 96.9%).
(3) MS (ESI): m/z [M+H]+theoretical value 413.2, measured value 413.1.

### 5. Synthesis of intermediate TO-25-01

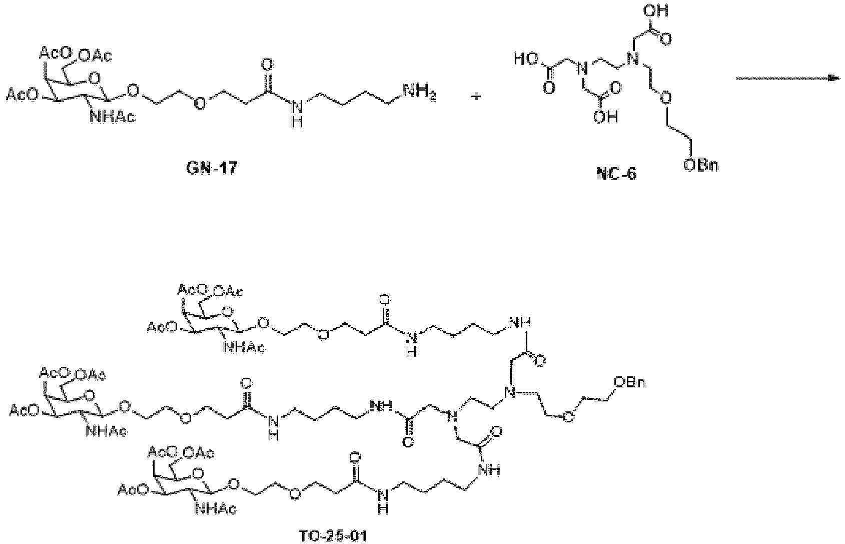

(1) Under the N₂ atmosphere, NC-6 (1.5 g, 3.6 mmol), HBTU (4.5 g, 12.0 mmol) and DIEA (4.75 g, 36 mmol) are added to DCM (50 mL) and stirred for 30 minutes, then DCM (50 mL) solution of GN-17 (6.4 g, 12.0 mmol) and DIEA (4.75 g, 36 mmol) are dropwise added, and stirred at 25°C for 16 hours. It is detected by a liquid chromatography mass spectrometry (LCMS) that raw materials are basically consumed.
(2) DCM (100 mL) is added for dilution, 1 N of hydrochloric acid solution (80 mL×2) is added to reaction solution for washing, organic phases are combined, and it is washed with the saturated sodium bicarbonate (100 mL), washed with the saturated salt water (100 mL), dried with the anhydrous Na₂SO₄, filtered and concentrated to obtain a crude product. The crude product is purified by the silica gel column chromatography (DCM/MeOH=7/1) to obtain a white solid compound TO-25-01 (4.3 g, yield: 60%).
(3) MS (ESI): m/z [M/2+H]+theoretical value 980.0, measured value 979.9.

### 6. Synthesis of intermediate TO-25

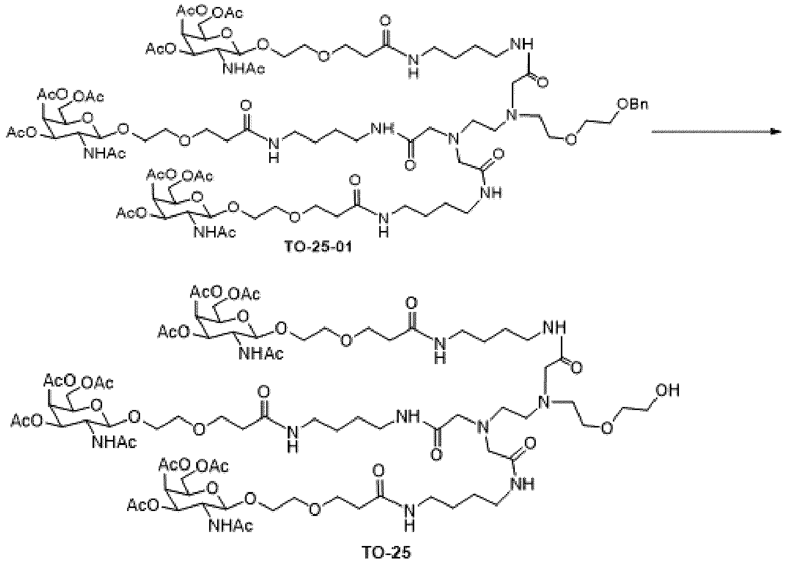

(1) TO-25-01 (4.3 g, 2.2 mmol) is dissolved in methanol (80 mL), 10% palladium carbon (1.0 g) is added, it is replaced with H₂ for three times, and stirred at 25°C for 2 hours. It is detected by LCMS that raw material are basically disappeared.
(2) It is filtered and concentrated, DCM (20 mL) is added to dissolve, it is slowly dripped into a methyl tert-butyl ether (MTBE) (300 mL), stirred and crystallized for 30 minutes, and suction-filtered, to obtain a white solid compound TO-25 (3.7 g, yield: 90%).

¹H NMR (400 MHz, DMSO-*d*₆) δ 8.48 (d, *J* = 5.6 Hz, 1H), 8.06 (t, *J* = 5.7 Hz, 2H), 7.85 (dd, *J* = 11.7, 6.8 Hz, 6H), 5.21 (d, *J* = 3.3 Hz, 3H), 4.95 (dd, *J* = 11.2, 3.3 Hz, 3H), 4.53 (d, *J* = 8.5 Hz, 3H), 4.08-3.83 (m, 14H), 3.75 (p, *J* = 4.8 Hz, 5H), 3.68-3.26 (m, 28H), 3.21-2.95 (m, 14H), 2.30 (q, *J* = 7.9, 6.7 Hz, 6H), 1.94-1.78 (m,, 36H), 1.41-1.38 (m, 12H); and

MS (ESI): m/z [1/2M+H]+theoretical value 934.9, measured value 934.8.

### 7. Synthesis of TP-25 (precursor of 1046 target linked to siRNA)

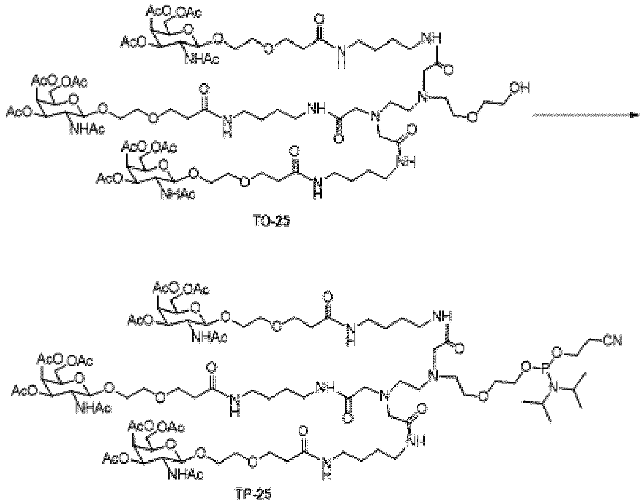

(1) Under the N₂ atmosphere, TO-25 (700 mg, 0.37 mmol) is dissolved in dry DCM (10 mL), DIEA (0.31 mL, 1.9 mmol) is added, dry DCM (1 mL) solution of 2-cyanoethyl-N,N-diisopropylchlorophosphoramidite (0.19 mL, 0.74 mmol) is slowly dripped with the injector, and it is reacted at 25°C for 30 minutes. It is detected by TLC that raw materials are basically disappeared.
(2) Saturated NaHCO₃ (10 mL) is added for quenching, it is diluted by DCM (10 mL), solution is separated, an organic phase is washed with saturated NaHCO₃ (10 mL) solution and saturated salt water (10 mL), it is dried with the anhydrous NaSO₄, filtered and concentrated to obtain a crude product. After the column chromatography purification (the silica gel column is alkalized by 1.5% TEA/DCM in advance, DCM/MeOH/TEA=15/1/0.1), a white solid TP-25 (405 mg, yield: 53%) is obtained.

¹H NMR (400 MHz, DMSO-*d*₆) δ 8.12 (t, *J* = 6.0 Hz, 2H), 7.98-7.75 (m, 7H), 5.21 (d, *J* = 3.4 Hz, 3H), 4.96 (dd, *J* = 11.2, 3.4 Hz, 2H), 4.54 (d, *J* = 8.4 Hz, 2H), 4.02 (q, *J* = 5.3, 4.5 Hz, 9H), 3.95-3.83 (m, 3H), 3.82-3.50 (m, 23H), 3.40-3.26 (m, 4H), 3.12-2.94 (m, 27H), 2.76-2.59 (m, 7H), 2.29 (t, *J* = 6.7 Hz, 5H), 2.11-1.78 (m, 38H), 1.38 (s, 12H), 1.16 (d, *J* = 7.5 Hz, 12H);

³¹P NMR (162 MHz, DMSO-d6) δ 147.97; and

MS (ESI): m/z [1/2M+Na]+theoretical value 1057.0, measured value 1057.4.

### III. Synthesis of GalNAc target 1048

According to the following method, a diastereoisomer of TO26 and TP-26 (a precursor of a 1048 target linked to siRNA) is synthesized.

### 1. Synthesis of intermediate GN-18-01

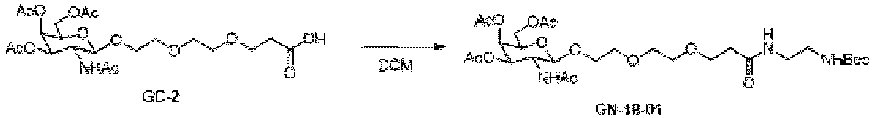

(1) Under the N₂ atmosphere, GC-2 (20.1 g, 39.7 mmol) is dissolved in DCM (200 mL), carbonyldiimidazole (CDI) (7.09 g, 73.7 mmol) is added in batches, it is stirred at 25°C for 3 hours, then N-Bocethylenediamine (7.0 g, 43.7 mmol) and triethylamine (12.05 g, 119.1 mmol) are added to reaction solution, and it is reacted for 16 hours. LCMS detection shows that raw materials are disappeared.
(2) The saturated sodium bicarbonate solution (200 mL) is added for quenching, solution is separated, a aqueous phase is extracted with DCM (100 mL×3), organic phases are combined, and it is washed with saturated ammonium chloride solution (200 mL) and saturated sodium chloride solution (200 mL), dried with the anhydrous Na₂SO₄, filtered and concentrated to obtain a crude product. The crude product is washed with the methyl tert-butyl ether (100 mL), and an oil-like product is concentrated to obtain a white solid compound GN-18-01 (24.43 g, yield: 95.1%).

MS (ESI): m/z [M+H]+theoretical value 650.3, measured value 650.5.

### 2. Synthesis of intermediate GN-18

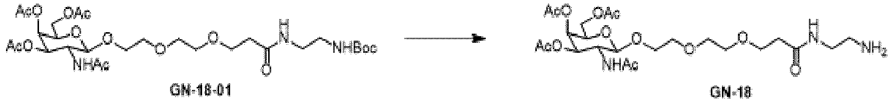

(1) GN-18-01 (45.52 g, 70 mmol) is added to HCl/EtOAc solution (2 N, 500 mL) in batches, and it is stirred at 25°C for 2 hours. LCMS detection shows that raw materials are disappeared.
(2) A solvent is poured out, and a solid is concentrated to obtain a crude product. The crude product is pulped and purified by the methyl tert-butyl ether (200 mL), it is filtered, and a filter cake is vacuum-dried at 40°C to obtain a white solid GN-18 (49.6 g).

MS (ESI): m/z [M+H]+theoretical value 550.3, measured value 550.5.

### 3. Synthesis of intermediate TO-26-01

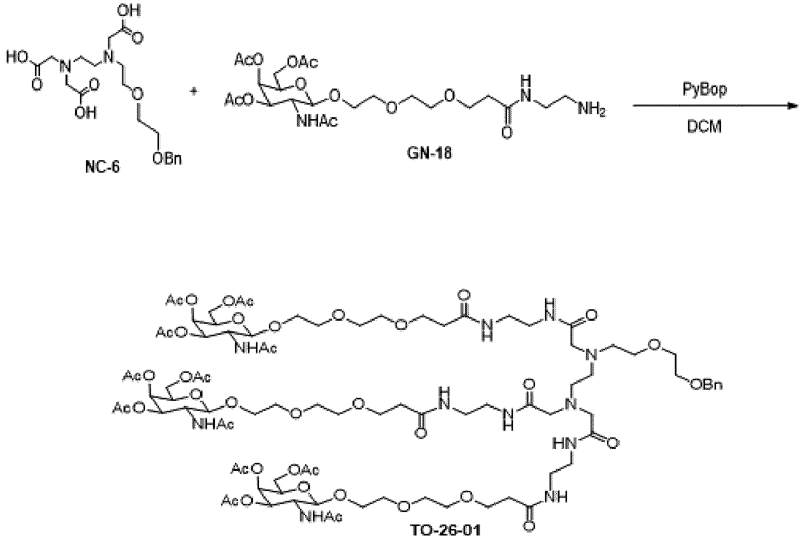

(1) Under the N₂ atmosphere, NC-6 (1.5 g, 3.6 mmol), hexafluorophosphate (PyBOP) (6.2 g, 12.0 mmol) and DIEA (4.75 g, 36 mmol) are added to DCM (50 mL) and stirred for 30 minutes, then DCM (50 mL) solution of GN-18 (6.6 g, 12.0 mmol) and DIEA (4.75 g, 36 mmol) is dropwise added, and it is stirred at 25°C for 16 hours. It is detected by LCMS that raw materials are basically consumed.
(2) DCM (100 mL) is added for dilution, 1 N of hydrochloric acid solution (80 mL×2) is added to reaction solution for washing, organic phases are combined, and it is washed with saturated sodium bicarbonate (100 mL) and saturated salt water (100 mL), dried with the anhydrous Na₂SO₄, filtered and concentrated to obtain a crude product. The crude product is purified by the silica gel column chromatography (DCM/MeOH=7/1) to obtain a white solid compound TO-26-01 (4.7 g, yield: 65%).

MS (ESI): m/z [M/2+H]+theoretical value 1004.0, measured value 1004.2.

### 4. Synthesis of intermediate TO-26

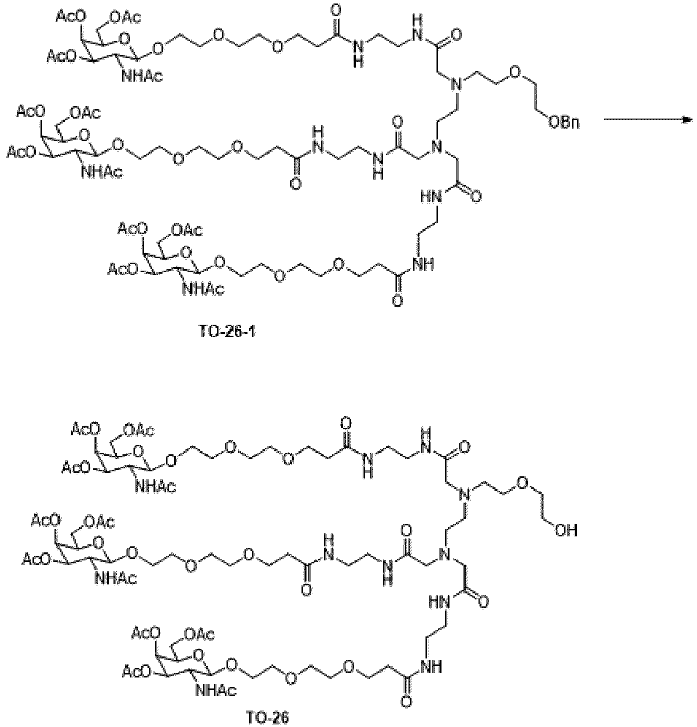

(1) TO-26-01 (4.0 g, 2.0 mmol) is dissolved in methanol (80 mL), 10% palladium carbon (1.0 g) is added, it is replaced with H₂ for three times, and stirred at 25°C for 2 hours. It is detected by LCMS that raw materials are basically disappeared.
(2) It is filtered, and concentrated, DCM (20 mL) is added to dissolve, it is slowly dripped into MTBE (200 mL), stirred and crystallized for 30 minutes, and suction-filtered, to obtain a white solid compound TO-26 (3.5 g, yield: 91%).

¹H NMR (400 MHz, DMSO-*d*₆) δ 8.54 (s, 1H), 8.14 (s, 2H), 7.95-7.92 (m, 3H), 7.84 (d, *J* = 7.8 Hz, 3H), 5.21 (d, *J* = 3.4 Hz, 3H), 4.97 (dd, *J* = 11.2, 3.4 Hz, 3H), 4.54 (d, *J* = 8.5 Hz, 3H), 4.13-3.66 (m, 21H), 3.60-3.44 (m, 37H), 3.14 (d, *J* = 13.8 Hz, 15H), 2.31 (t, *J* = 6.4 Hz, 6H), 2.10 (s, 9H), 2.00 (s, 9H), 1.89 (s, 9H), 1.77 (s, 9H).

MS (ESI): m/z [1/2M+H]+theoretical value 958.9, measured value 959.1.

### 5. Synthesis of TP-26 (precursor of 1048 target linked to siRNA)

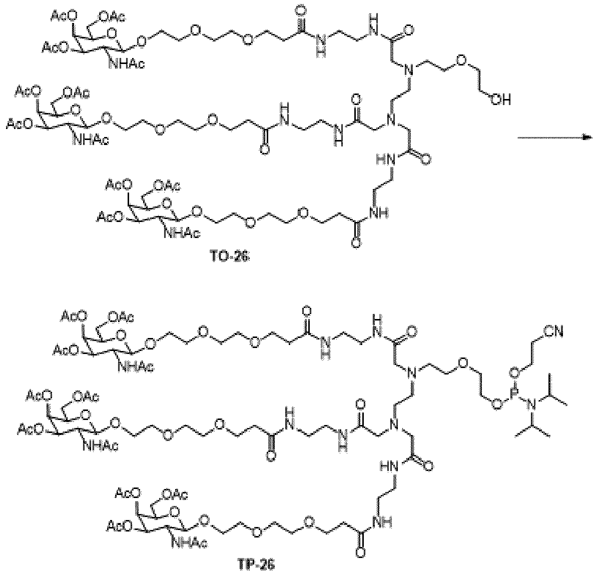

(1) Under the N₂ atmosphere, TO-26 (900 mg, 0.47 mmol) is dissolved in dry DCM (12 mL), DIEA (0.39 mL, 0.44 mmol) is added, dry DCM (1 mL) solution of 2-cyanoethyl-N, N-diisopropylchlorophosphoramidite (277 mg, 1.17 mmol) is slowly dripped with the injector, and it is reacted at 25°C for 30 minutes. It is detected by TLC that raw materials are basically disappeared.
(2) Saturated NaHCO₃ (10 mL) is added for quenching, it is diluted by DCM (10 mL), solution is separated, an organic phase is washed with saturated NaHCO₃ (10 mL) solution and saturated salt water (10 mL), dried with the anhydrous NaSO₄, filtered and concentrated to obtain a crude product. After the column chromatography purification (the silica gel column is alkalized by 1.5% TEA/DCM in advance, DCM/MeOH/TEA=15/1/0.1), a white solid TP-26 (600 mg, yield: 60%) is obtained.

¹H NMR (400 MHz, DMSO-d*6*) ¹H NMR (400 MHz, DMSO-*d*₆) δ 8.15 (s, 2H), 7.94-7.81 (m, 7H), 5.22 (d, *J* = 3.4 Hz, 3H), 4.97 (dd, *J* = 11.2, 3.4 Hz, 3H), 4.55 (d, *J* = 8.5 Hz, 3H), 4.03 (s, 8H), 3.88 (dt, *J* = 11.2, 8.9 Hz, 3H), 3.81- 3.67 (m, 7H), 3.64 - 3.46 (m, 30H), 3.11 (d*, J* = 13.1 Hz, 19H), 2.76 (t, *J* = 5.9 Hz, 3H), 2.65 - 2.54 (m, 7H), 2.31 (t, *J* = 6.6 Hz, 7H), 2.11 (s, 9H), 2.00 (s, 9H), 1.89 (s, 9H), 1.77 (s, 9H), 1.13 (d, *J* = 6.8, 12H).

³¹P NMR (162 MHz, DMSO-*d*6) δ 147.89; and

MS (ESI): m/z 1/2 [M-i-Pr₂N] theoretical value 1007.9, measured value 1008.2.

### Embodiment 3: In vitro construction of siRNA conjugate coupled (modified) by coupling GalNAc target

Oligonucleotide sequence portions of an antisense chain and a sense chain of the following RNAi agent double-chain body, as well as linkage between a target ligand and RNA, are all in accordance with phosphite amide coupling technologies reported by J Org. Chem. 2012, 77, 4566-4577; Curr. Protoc. Nucleic Acid Chem., 81, e107, and are synthesized on a solid phase for oligonucleotide synthesis. The target ligands 1046, 1048 and 1043 are all linked to a 5'-end of the siRNA sense chain by a thiophosphate bond.

The form of the target ligands 1046, 1048 and 1043 linked with siRNA is a form of removing a hydroxyl protecting group, and it is specifically as follows:

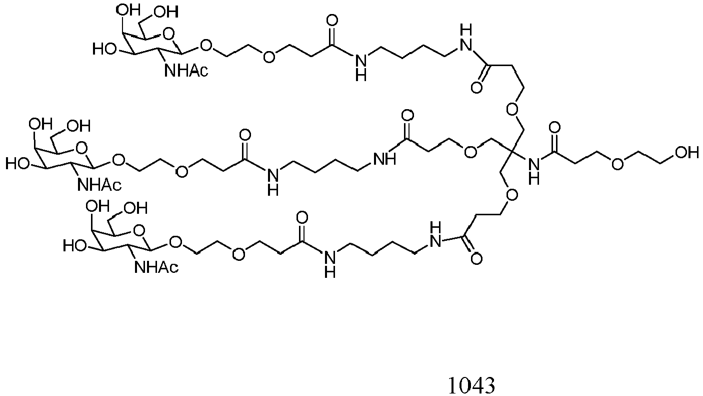

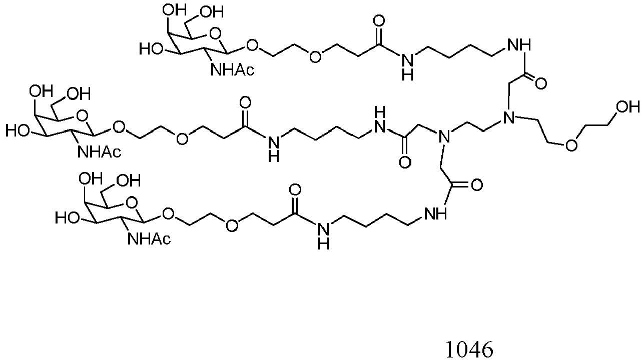

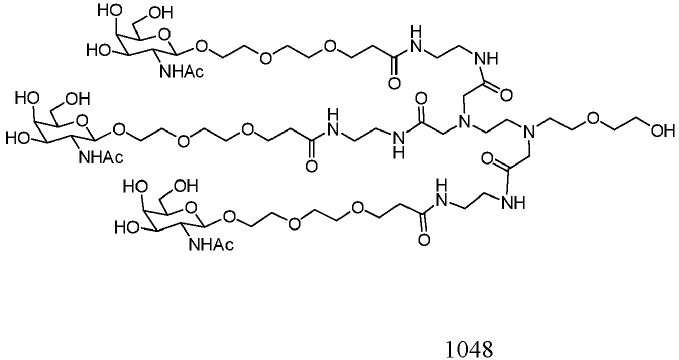

The structures of the target ligands after being linked with siRNA are as follows:

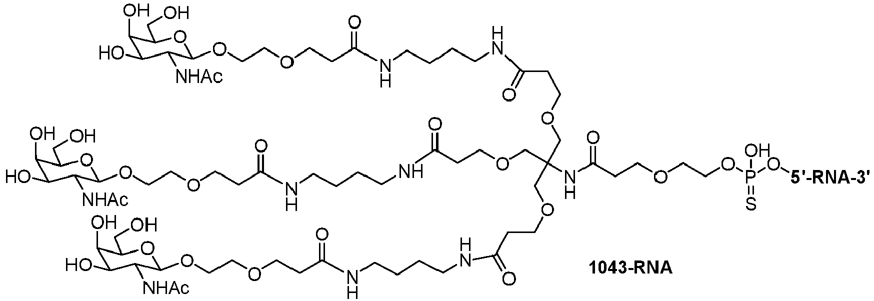

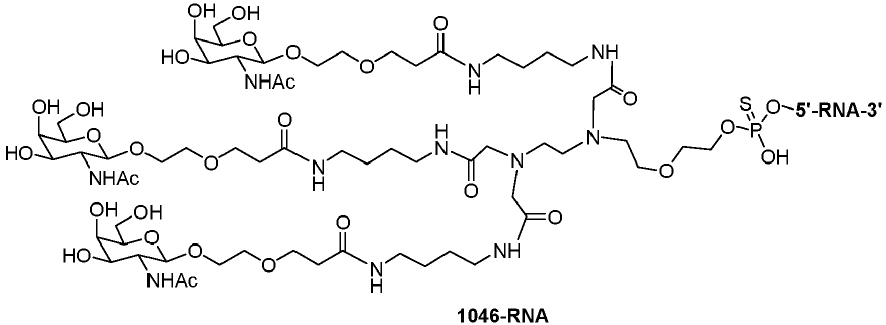

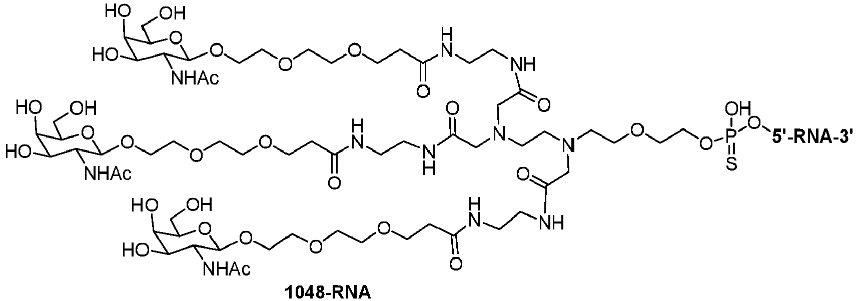

The synthesized GalNAc-siRNA conjugate is described in Fig. 3, herein the structure of the conjugate in the second column includes three portions. For example, the structure of G1043-S2A2-A265 is: the 1043 target is linked with the 5'-end of the siRNA sense chain numbered as A265 by the thiophosphate bond, and S2A2 is the type of modification to siRNA of A265. The specific modification groups and modification modes are as follows.

In the nucleic acid sequence, Ao represents an adenosine, Uo represents a uridine, Go represents a guanosine, and Co represents a cytosine, and there is no symbol between directly adjacent nucleotides, it is indicated that it is linked by a normal phosphate bond.

DNA: A, G, C, T (A represents 2'-deoxyadenosine, T represents 2'-deoxythymidine, G represents 2'-deoxyguanosine, and C represents 2'-deoxycytidine).

2'-F: aF, gF, cF, uF (aF represents 2'-fluoroadenine nucleoside, uF represents 2'-fluorouracil nucleoside, gF represents 2'-fluoroguanine nucleoside, and cFrepresents 2'-fluorocytosine nucleoside).

2'-OMe: aM, gM, cM, uM (aM represents 2'-O-methyladenine nucleoside, uM represents 2'-O-methyluracil nucleoside, gM represents 2'-O-methylguanine nucleoside, and cM represents 2'-O-methylcytosine nucleoside).
*: represents that it is linked by a thiophosphate bond.
y and z in the sequence represent the position of the target.

### Embodiment 4: Activity test of conjugate by in vitro cell model (Hep 3B cell)

A human hepatoma Hep3B cell (Shanghai Cell Bank, Chinese Academy of Sciences) is cultured in DMEM (Gibco, US) supplemented with 10% FBS (Gibco, US) under conditions of 37°C and 5% CO₂ (il60, Thermo Fisher). On the day of a transfection experiment, the cells are digested with 0.25% Trysin (Gibco, US), counted and inoculated on a 24-well plate in the density of 450 µL/well and 50000 cells/well. Subsequently, a test sample is added in a lipofectmine2000 (Thermo Fisher) transfection mode. It is transfected according to a standard flow of RNAiMAX reagent instructions, and the final siRNA concentration is 10 nM/1 nM/0.5 nM/0.25 nM/0.1 nM/0.05 nM/0.01 nM. In a transfection group, siNC is taken as a negative control, and its sequence is as follows.
Sense chain (sense): 5'-UUCCGAACGUGUCACGUTT-3'(SEQ ID NO.309),
Antisense chain (antisense): 5'-ACGUGACACGUUCGGAGAATT-3'(SEQ ID NO.310).

After 24 h, the total RNA of the cells is extracted, and the expression conditions of the ANGPTL3 mRNA sequence in the cells are detected by the quantitative real-time PCR, herein PCR primers used to amplify internal reference genes PPIB and ANGPTL3 are shown in Table 1.

The activity test results (EC₅₀ value) of each conjugate are shown in Fig. 4.

The EC₅₀ value is calculated by using non-linear regression of graphpad prism, to express the amount of the conjugate used to inhibit a half of the expression quantity of the target ANGPTL3mRNA.

It may be seen from the results that the selected conjugates show good results in reducing the relative expression level of ANGPTL3 in an experiment of the in vitro activity test.

### Embodiment 5: Construction of AAV-hANGPTL3 mouse model and drug administration test

Basic information of experimental animals:
The experimental animals are purchased from Jinan Pengyue Experimental Animal Breeding Co., Ltd., which are specific pathogen free (SPF) animals. Before drug administration, the above mice are weighed and statuses are observed, and the animals with uniform weight and no abnormal status are selected for subsequent experiments.

| Species | Gender | Age | Weight | Source |
|---|---|---|---|---|
| C57 mouse | Male | 4 weeks | 20±2 g | Jinan Pengyue |

Feeding conditions: non-SPF feeding conditions. Under normal feeding conditions, the animals may eat and drink freely. After the animals are purchased, the experiment is started after 3-7 days of adaptive culture.

Modeling and administration: each mouse is injected with 2.5^{∗}10^11 titers of virus solution (100 µ L) by a tail vein. After 7 days, the experimental animals are randomly grouped, and each test substance is administered subcutaneously at a dose of 5 mg/kg. In 72 hours after the drug administration, the animals are sacrificed by cervical dislocation, and liver tissues are taken for RNA extraction and quantification.

Results of each conjugate are shown in Table 3.

**Table 3: Drug administration test results of mouse model for each conjugate**

| **Test substance** | **hANGPTL3relative expression level** | | |
|---|---|---|---|
| | **Average value** | **Standard deviation** | **N (number of animals)** |
| PBS control | 1 | 0.24 | 6 |
| NPD006s-129 | 0.47 | 0.12 | 6 |
| NPD006s-130 | 0.44 | 0.22 | 5 |
| NPD006s-131 | 0.34 | 0.07 | 4 |
| NPD006s-132 | 0.41 | 0.16 | 5 |
| NPD006s-133 | 0.45 | 0.15 | 6 |
| NPD006s-134 | 0.55 | 0.12 | 5 |
| NPD006s-135 | 0.79 | 0.2 | 5 |
| NPD006s-136 | 0.55 | 0.14 | 6 |
| NPD006s-137 | 0.47 | 0.17 | 4 |
| NPD006s-138 | 0.61 | 0.43 | 5 |
| NPD006s-139 | 0.74 | 0.09 | 5 |
| NPD006s-140 | 0.35 | 0.11 | 5 |
| NPD006s-141 | 0.52 | 0.28 | 5 |
| NPD006s-143 | 0.6 | 0.09 | 5 |
| NPD006s-144 | 0.52 | 0.07 | 5 |
| NPD006s-145 | 0.46 | 0.07 | 4 |
| NPD006s-146 | 0.62 | 0.25 | 5 |
| NPD006s-147 | 0.39 | 0.12 | 5 |
| NPD006s-148 | 0.51 | 0.14 | 5 |
| NPD006s-151 | 0.40 | 0.31 | 5 |
| NPD006s-167 | 0.47 | 0.14 | 5 |
| NPD006s-168 | 0.47 | 0.26 | 5 |
| NPD006s-169 | 0.58 | 0.29 | 5 |

It may be seen from the results that the selected conjugates also show the good results in reducing the relative expression level of ANGPTL3 in the experiment of the in vivo activity test.

In descriptions of this description, the descriptions of reference terms such as "one embodiment", "some embodiments", "examples", "specific examples", or "some examples" means that specific features, structures, materials, or characteristics described in combination with this embodiment or example are included in at least one embodiment or example of the present disclosure. In this description, the schematic expressions of the above terms need not refer to the same embodiments or examples. Furthermore, the specific features, structures, materials, or characteristics described may be combined in an appropriate manner in any one or more embodiments or examples. In addition, those skilled in the art may incorporate and combine different embodiments or examples described in this description and the characteristics of the different embodiments or examples in the case without conflicting.

Although the embodiments of the present disclosure are already shown and described above, it may be understood that the above embodiments are exemplary, and may not be understood as limitation to the present disclosure. Those of ordinary skill in the art may change, modify, replace and transform the above embodiments within the scope of the present disclosure.

## Claims

1. A compound, wherein the compound has any one of the following structures:

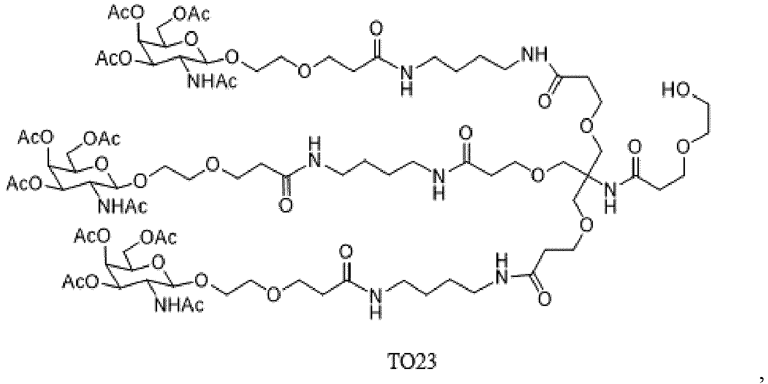

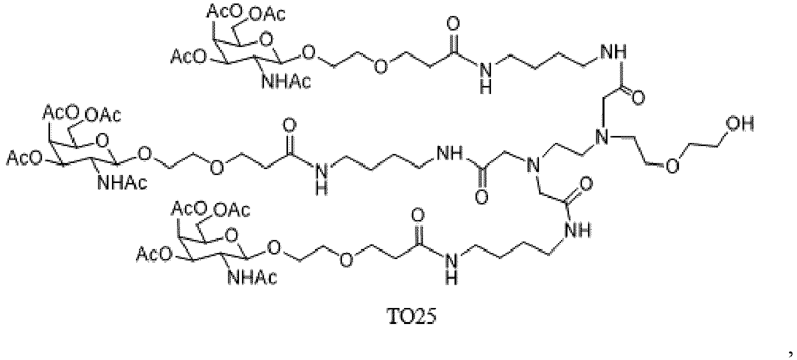

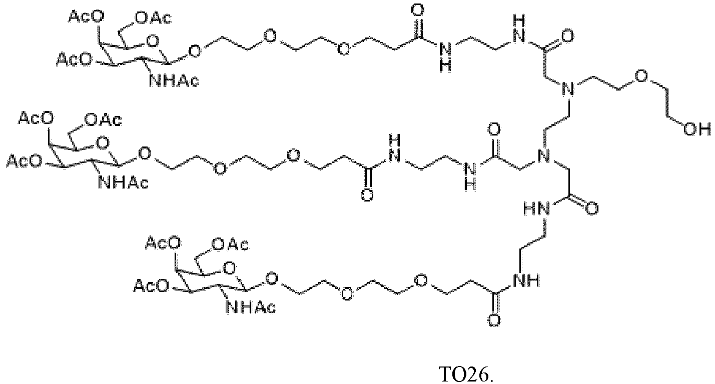

2. A compound, wherein the compound is obtained from the compound according to claim 1 by removing a hydroxyl protecting group, and has one of the following structures:

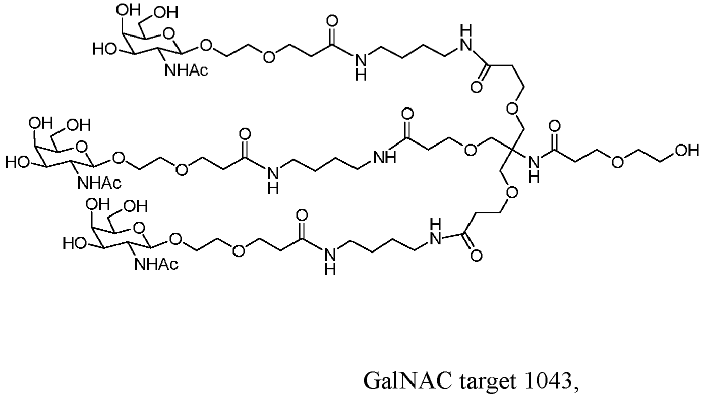

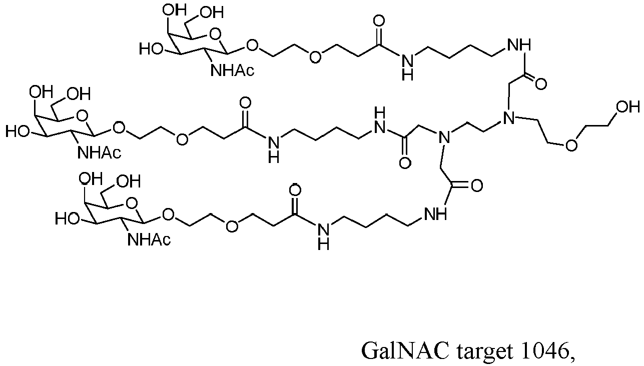

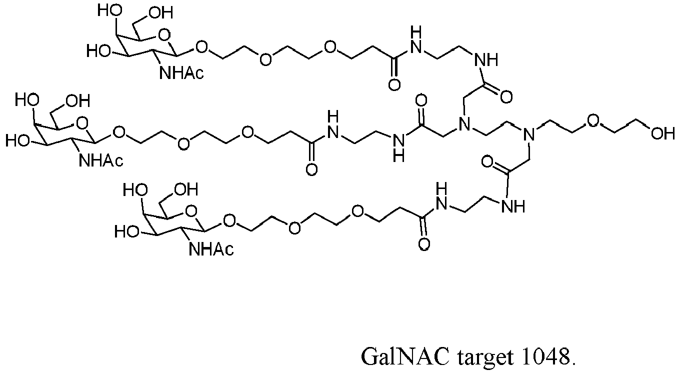

3. An application of the compound according to any one of claims 1-2 in preparation of a siRNA conjugate.

4. The application according to claim 3, wherein the compound is linked with siRNA as a target ligand.

5. The application according to claim 4, wherein siRNA in the siRNA conjugate comprises a sense chain and an antisense chain, and the antisense chain comprises a complementary region complementary-paired to the sense chain, wherein the sense chain is selected from a nucleotide sequence that is not more than 5 nucleotides different from a nucleotide sequence of each chain in SEQ ID NO: 1-SEQ ID NO: 154, and the antisense chain is selected from a nucleotide sequence that is not more than 5 nucleotides different from a nucleotide sequence of each chain in SEQ ID NO: 155-SEQ ID NO: 308.

6. The application according to claim 5, wherein the siRNA comprises at least one modified nucleotide;
the modified nucleotide is selected from at least one of the following:
a 5'-thiophosphate based nucleotide, a 5-methylcytosine nucleotide, a 2'-O-methyl modified nucleotide, a 2'-O-2-methoxyethyl modified nucleotide, a 2'-fluoro modified nucleotide, a 3'-nitrogen substituted modified nucleotide, a 2'-deoxy-2'-fluoro modified nucleotide, a 2'-deoxy modified nucleotide, a locked nucleotide, a de-base nucleotide, a 2'-amino modified nucleotide, a morpholinonucleotide, a polypeptide nucleotide, an amino phosphate, and a nucleotide comprising a non-natural base.

7. The application according to claim 6, wherein the siRNA is covalently linked with the target ligand; the target ligand is linked to the sense chain in the siRNA; and the target ligand is linked with a 5'-end of the sense chain in the siRNAby a thiophosphate bond.

8. A preparation method for asiRNA conjugate, comprising using the compound according to any one of claims 1-2as a target ligand to covalently link with siRNA.

9. The method according to claim 8, wherein the compound firstly forms an intermediate and then covalently links with siRNA, and the intermediate is selected from one of the following structures:

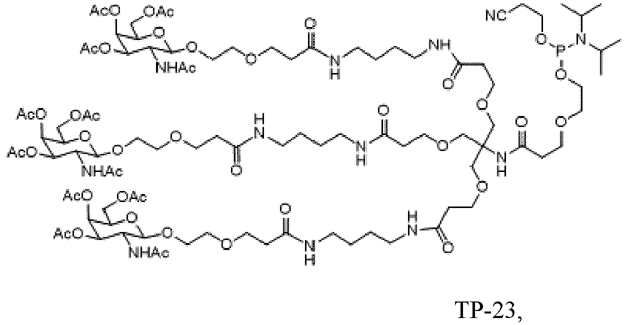

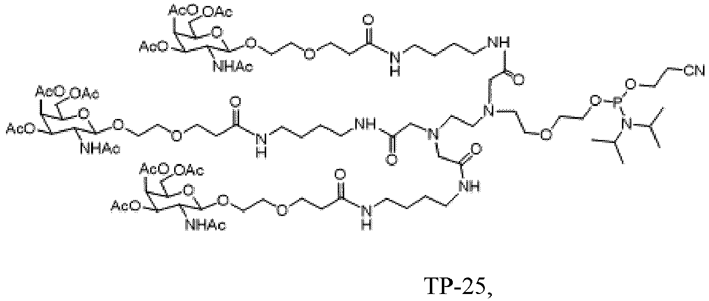

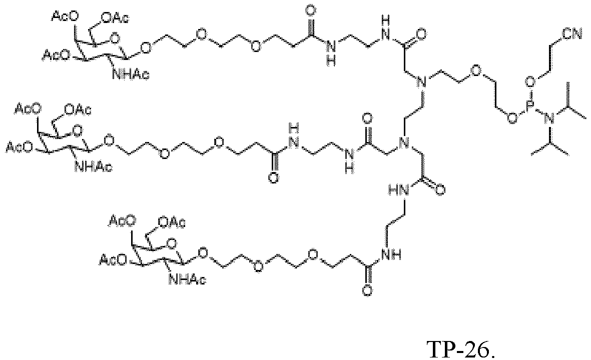

10. An intermediate for preparing a siRNA conjugate, wherein it is selected from one of the following structures:

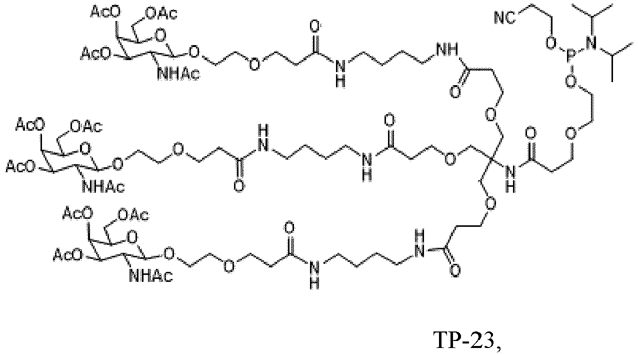

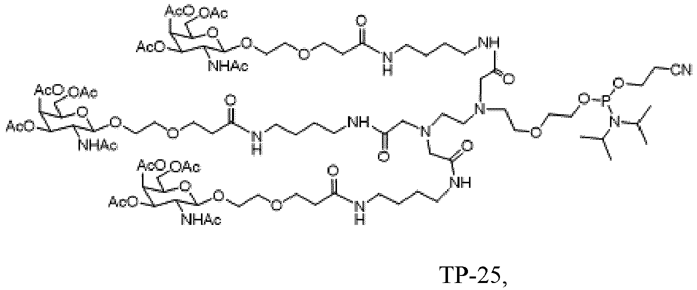

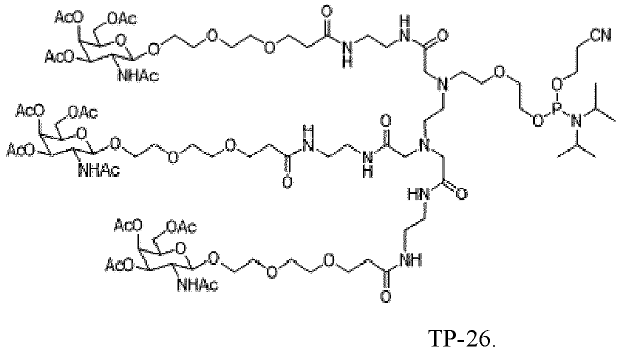

11. An application of the compound according to any one of claims 1-2 and/or the intermediate according to claim 10 in preparation of a drug or kit, wherein the drug or kit is used to inhibit the expression of the *ANGPTL3* gene.

12. The application according to claim 11, wherein the drug or kit is used to prevent and/or treat a dyslipidemia disease.

13. The application according to claim 12, wherein the dyslipidemia disease includes hyperlipidemia and hypertriglyceridemia.
